(19) Europäisches Patentamt
European Patent Office
Office européen des brevets

(11) Publication number: **0 318 143 A2**

(12) **EUROPEAN PATENT APPLICATION**

(21) Application number: 88309438.5

(22) Date of filing: 10.10.88

(51) Int. Cl.4: C12N 15/00 , C12P 21/02 , C12N 1/20 , //(C12N1/20, C12R1:07)

The microorganism(s) has (have) been deposited with Nothern Regional Research Laboratory under number(s) NRRL B-18257, B-18258.

(30) Priority: **13.10.87 US 108285**

(43) Date of publication of application: **31.05.89 Bulletin 89/22**

(84) Designated Contracting States: **AT BE CH DE ES FR GB GR IT LI LU NL SE**

(71) Applicant: **LUBRIZOL GENETICS, INC.**
**29400 Lakeland Boulevard**
**Wickliffe Ohio 44092(US)**

(72) Inventor: **Sekar, Vaithlingham Department of Biotechnology**
**Madurai Kamari University Madurai 625021**
**Tami Nadu(IN)**
Inventor: **Adang, Michael J.**
**601 Valley Road**
**Madison Wisconsin 53714(US)**

(74) Representative: **Fisher, Adrian John et al**
**CARPMAELS & RANSFORD 43 Bloomsbury Square**
**London WC1A 2RA(GB)**

(54) **Anti-coleopteran toxin and gene.**

(57) The present invention relates to a novel biocontrol protein which is a crystal toxin and an insecticide and which is produced in a strain of bacteria genetically altered upon incorporation of a specific gene inserted as a stable gene construction. In particular, the present invention relates to the insertion of the δ-endotoxin gene from B. thuringiensis var. tenebrionis into a cloning vector containing prokaryotic regulatory and replication systems and the utilization of this vector to transform bacterial strains into coleopteran pathogens. Limited deletion of the coding region of the gene of this invention still allows preservation of the insecticidal activity in the protein expressed. In addition, this invention provides genetically altered bacterial strains that are insect toxic and that can be used as a novel method of biological control of agricultural insect pests.

FIG. 2-I

```
                                                                                           Ec→                                          Bt→
601  TTAAATGAATATGTAAATATATTTATGATAAGAAGCGACTTATTTATAATCATTACATATTTTTCTATTGGAATGATTAAGATTCCAATAGAATAGTGTATAAATTATTTATCTTGAAAG
721  GAGGGATGCCTAAAAACGAAGAACATTAAAAACATATATTTGCACCGTCTAATGGATTTATGAAAAATCATTTTATCAGTTTGAAAATTATGTATTATGATAAGAAAGGGAGGAAGAAAA
841  ATGAATCCGAACAATCGAAGTGAACATGATACAATAAAAACTACTGAAAATAATGAGGTGCCAACTAACCATGTTCAATATCCTTTAGCGGAAACTCCAAATCCAACACTAGAAGATTTA
     MetAsnProAsnAsnArgSerGluHisAspThrIleLysThrThrGluAsnAsnGluValProThrAsnHisValGlnTyrProLeuAlaGluThrProAsnProThrLeuGluAspLeu
961  AATTATAAAGAGTTTTTAAGAATGACTGCAGATAATAATACGGAAGCACTAGATAGCTCTACAACAAAAGATGTCATTCAAAAAGGCATTTCCGTAGTAGGTGATCTCCTAGGCGTAGTA
     AsnTyrLysGluPheLeuArgMetThrAlaAspAsnAsnThrGluAlaLeuAspSerSerThrThrLysAspValIleGlnLysGlyIleSerValValGlyAspLeuLeuGlyValVal
1081 GGTTTCCCGTTTGGTGGAGCGCTTGTTTCGTTTTATACAAACTTTTTAAATACTATTTGGCCAAGTGAAGACCCGTGGAAGGCTTTTATGGAACAAGTAGAAGCATTGATGGATCAGAAA
     GlyPheProPheGlyGlyAlaLeuValSerPheTyrThrAsnPheLeuAsnThrIleTrpProSerGluAspProTrpLysAlaPheMetGluGlnValGluAlaLeuMetAspGlnLys
1201 ATAGCTGATTATGCAAAAAATAAAGCTCTTGCAGAGTTACAGGGCCTTCAAAATAATGTCGAAGATTATGTGAGTGCATTGAGTTCATGGCAAAAAAATCCTGTGAGTTCACGAAATCCA
     IleAlaAspTyrAlaLysAsnLysAlaLeuAlaGluLeuGlnGlyLeuGlnAsnAsnValGluAspTyrValSerAlaLeuSerSerTrpGlnLysAsnProValSerSerArgAsnPro
1321 CATAGCCAGGGGCGGATAAGAGAGCTGTTTTCTCAAGCAGAAAGTCATTTTCGTAATTCAATGCCTTCGTTTGCAATTTCTGGATACGAGGTTCTATTTCTAACAACATATGCACAAGCT
     HisSerGlnGlyArgIleArgGluLeuPheSerGlnAlaGluSerHisPheArgAsnSerMetProSerPheAlaIleSerGlyTyrGluValLeuPheLeuThrThrTyrAlaGlnAla
1441 GCCAACACACATTTATTTTTACTAAAAGACGCTCAAATTTATGGAGAAGAATGGGGATACGAAAAAGAAGATATTGCTGAATTTTATAAAAGACAACTAAAACTTACGCAAGAATATACT
     AlaAsnThrHisLeuPheLeuLeuLysAspAlaGlnIleTyrGlyGluGluTrpGlyTyrGluLysGluAspIleAlaGluPheTyrLysArgGlnLeuLysLeuThrGlnGluTyrThr
1561 GACCATTGTGTCAAATGGTATAATGTTGGATTAGATAAATTAAGAGGTTCATCTTATGAATCTTGGGTAAACTTTAACCGTTATCGCAGAGAGATGACATTAACAGTATTAGATTTAATT
     AspHisCysValLysTrpTyrAsnValGlyLeuAspLysLeuArgGlySerSerTyrGluSerTrpValAsnPheAsnArgTyrArgArgGluMetThrLeuThrValLeuAspLeuIle
1681 GCACTATTTCCATTGTATGATGTTCGGCTATACCCAAAAGAAGTTAAAACCGAATTAACAAGAGACGTTTTAACAGATCCAATTGTCGGAGTCAACAACCTTAGGGGCTATGGAACAACC
     AlaLeuPheProLeuTyrAspValArgLeuTyrProLysGluValLysThrGluLeuThrArgAspValLeuThrAspProIleValGlyValAsnAsnLeuArgGlyTyrGlyThrThr
```

EP 0 318 143 A2

```
1801 TTCTCTAATATAGAAAATTATATTCGAAAACCACATCTATTTGACTATCTGCATAGAATTCAATTTCACACGCGGTTCCAACCAGGATATTATGGAAATGACTCTTTCAATTATTGGTCC
     PheSerAsnIleGluAsnTyrIleArgLysProHisLeuPheAspTyrLeuHisArgIleGlnPheHisThrArgPheGlnProGlyTyrTyrGlyAsnAspSerPheAsnTyrTrpSer

1921 GGTAATTATGTTTCAACTAGACCAAGCATAGGATCAAATGATATAATCACATCTCCATTCTATGGAAATAAATCCAGTGAACCTGTACAAAATTTAGAATTTAATGGAGAAAAAGTCTAT
     GlyAsnTyrValSerThrArgProSerIleGlySerAsnAspIleIleThrSerProPheTyrGlyAsnLysSerSerGluProValGlnAsnLeuGluPheAsnGlyGluLysValTyr

2041 AGAGCCGTAGCAAATACAAATCTTGCGGTCTGGCCGTCCGCTGTATATTCAGGTGTTACAAAAGTGGAATTTAGCCAATATAATGATCAAACAGATGAAGCAAGTACACAAACGTACGAC
     ArgAlaValAlaAsnThrAsnLeuAlaValTrpProSerAlaValTyrSerGlyValThrLysValGluPheSerGlnTyrAsnAspGlnThrAspGluAlaSerThrGlnThrTyrAsp

2161 TCAAAAAGAAATGTTGGCGCGGTCAGCTGGGATTCTATCGATCAATTGCCTCCAGAAACAACAGATGAACCTCTAGAAAAGGGATATAGCCATCAACTCAATTATGTAATGTGCTTTTTA
     SerLysArgAsnValGlyAlaValSerTrpAspSerIleAspGlnLeuProProGluThrThrAspGluProLeuGluLysGlyTyrSerHisGlnLeuAsnTyrValMetCysPheLeu

2281 ATGCAGGGTAGTAGAGGAACAATCCCAGTGTTAACTTGGACACATAAAAGTGTAGACTTTTTTAACATGATTGATTCGAAAAAAATTACACAACTTCCGTTAGTAAAGGCATATAAGTTA
     MetGlnGlySerArgGlyThrIleProValLeuThrTrpThrHisLysSerValAspPhePheAsnMetIleAspSerLysLysIleThrGlnLeuProLeuValLysAlaTyrLysLeu

2401 CAATCTGGTGCTTCCGTTGTCGCAGGTCCTAGGTTTACAGGAGGAGATATCATTCAATGCACAGAAAATGGAAGTGCGGCAACTATTTACGTTACACCGGATGTGTCGTACTCTCAAAAA
     GlnSerGlyAlaSerValValAlaGlyProArgPheThrGlyGlyAspIleIleGlnCysThrGluAsnGlySerAlaAlaThrIleTyrValThrProAspValSerTyrSerGlnLys

2521 TATCGAGCTAGAATTCATTATGCTTCTACATCTCAGATAACATTTACACTCAGTTTAGACGGGGCACCATTTAATCAATACTATTTCGATAAAACGATAAATAAAGGAGACACATTAACG
     TyrArgAlaArgIleHisTyrAlaSerThrSerGlnIleThrPheThrLeuSerLeuAspGlyAlaProPheAsnGlnTyrTyrPheAspLysThrIleAsnLysGlyAspThrLeuThr

2641 TATAATTCATTTAATTTAGCAAGTTTCAGCACACCATTCGAATTATCAGGGAATAACTTACAAATAGGCGTCACAGGATTAAGTGCTGGAGATAAAGTTTATATAGACAAAATTGAATTT
     TyrAsnSerPheAsnLeuAlaSerPheSerThrProPheGluLeuSerGlyAsnAsnLeuGlnIleGlyValThrGlyLeuSerAlaGlyAspLysValTyrIleAspLysIleGluPhe

                                                                                                  ---------------------------------> IR
2761 ATTCCAGTGAATTAAATTAACTAGAAAGTAAAGAAGTAGTGACCATCTATGATAGTAAGCAAAGGATAAAAAAATGAGTTCATAAAATGAATAACATAGTGTTCTTCAACTTTCGCTTTT
     IleProValAsnEnd
                <------------------------------ IR
2881 TGAAGGTAGATGAAGAACACTATTTTTATTTTCAAAATGAAGGAAGTTTTAAATATGTAATCATTTAAAGGGAACAATGAAAGTAGGAAATAAGTCATTATCTATAACAAAATAACATTT
```

FIG. 2-2

## ANTI-COLEOPTERAN TOXIN AND GENE

### FIELD OF THE INVENTION

This invention relates to the field of bacterial molecular biology and, in particular, to genetic engineering by recombinant technology for the purpose of protecting plants from coleopteran insect pests. Disclosed herein are the cloning, characterization and the selective expression of the intact as well as partially modified δ-endotoxin gene from B. thuringiensis var. tenebrionis. Also disclosed is the transfer of the cloned gene into a host microorganism, rendering the organism able to produce a protein having toxicity to coleopteran insects. This invention will facilitate the genetic engineering of bacteria to express novel toxins having agronomic value.

### BACKGROUND OF THE INVENTION

Of the bacteria pathogenic to insects, the spore forming bacilli - of which Bacillus thuringiensis forms a major group - are viewed as having the highest potential for use in the management of insect pest populations. B. thuringiensis produces protoxins during sporulation. These proteins are deposited as parasporal inclusions or on the surface of the spore. The toxic action occurs when the inclusions or spores are ingested by susceptible larvae. A mechanism for the toxic effects is not known in detail, but some of the steps are known. After ingestion, the protoxins are solubilzed in the midgut where pH conditions and proteases convert the protoxins to toxins, which then bind to receptor molecules on the surface of midgut epithelial cells, causing disruption of midgut structure and function and eventual death. Several B. thuringiensis toxins have been described, having a range of activities, including primarily many species of Lepidoptera, some Diptera and, recently discovered, species of Coleoptera.

One advantage of using B. thuringiensis for practical pest control is the production of stable spores which are readily formulated for use in conventional pest control application equipment and which are safe for human, other mammal and nontarget fauna. However, problems exist because B. thuringiensis spores and parasporal crystals applied to foliage do not persist in natural environments, being washed off by rain and inactivated by sunlight so that only relatively short term protection from pest populations is obtained from single applications. Thus, when B. thuringiensis is used as a conventional insecticidal poison, it must be applied repeatedly for long term protection of agricultural crops. Moreover, while there are available isolates of B. thuringiensis that have broad spectrum toxicity for lepidopteran pests, no single isolate is active against all pest species.

Classification of the many subspecies of B. thuringiensis has been attempted on the basis of flagella serotype and crystal serotype (H.T. Dulmage (1981) in Microbial Control of Pests and Plant Diseases, H.D. Burges (ed.), 1970-1980, Academic Press, London, pp. 193-222). For the most part, there appeared a correlation between flagella (H) serotype and crystal serotype (for example, most B. thuringiensis var. thuringiensis (serotype H-1) isolates were of the thu crystal type). An exception to this predominance of one crystal type in each serological group was found among the B. thuringiensis var. kurstaki isolates which were divided into two crystal types (k-1 and k-73 types). The k-1 and k-73 type isolates were also distinguished by different activity spectra. Other exceptions were also observed. Serologically identical crystals appeared in different flagella serotypes; for example, k-1 crystals were present in both kurstaki and thuringiensis subspecies. Furthermore, some isolates contained a mixed crystal type, indicating either that one isolate may contain two protoxins or that a given protoxin may share both major antigenic determinants (J. Krywienczyk et al. (1978) J. Invertebr. Pathol. 31:372-375; (1981) J. Invertebr. Pathol. 37:62-65). It was found that the k-1 crystal type from the kurstaki subspecies contains two distinct toxins, the major lepidopteran protoxin (δ-endotoxin or P1) and a minor toxin (P2) active on both Lepidoptera and Diptera. K-1 type crystals are also present in other subspecies and it is possible that both P1 and P2 will be found in these non-kurstaki isolates.

Crystal types have been further subdivided on the basis of activity spectra. Dulmage (1981), supra, examined the activity spectra of 38 isolates containing thu-type crystals and 36 containing the k-1 types. The 38 thu preparations could be divided into 13 activity groups, the largest consisting of 10 isolates. Similarly, the 36 k-1 preparations were divided into 19 groups, the largest having four isolates. These subgroupings emphasize once again the complexity and variability of the B. thuringiensis crystals in that two isolates of the same crystal serotype may differ significantly in activity spectrum. A possible explanation is that there has been exchange of plasmids (or protoxin genes or both) among these natural isolates. It has been shown that there is transfer of protoxin encoding plasmids by cell mating and strains producing two different parasporal antigens have been constructed (J. Gonzales et al. (1982) Proc. Natl. Acad. Sci. 79:6951-6955; A. Klier et al. (1983) Mol. Gen. Genet. 191:257-262; B. Carlton and J. Gonzales, Jr. (1984) in Genetics and Biotechnolooy of Bacilli, A. T. Ganesan and J.A. Hoch (eds.), Academic Press, Inc., New York, pp. 387- 400). Such exchanges may occur naturally and contribute to new types via the presence of two or more different protoxin genes or even recombination between two such genes to create a new species of protoxin. It is now clear that a given B. thuringiensis strain may produce more than one protoxin and even crystals of a given strain may contain more than one protoxin. The activity spectra and immunological properties of these protoxins may differ from one another. Two new strains of B. thuringiensis have been reported recently - namely, var. tenebrionis (Krieg et al. (1983) Zschr. Angew. Entomol. 96:500-508) and var. san diego (Herrnstadt et al. (1986) Bio/Technology 4:305-308). Both subspecies are pathogenic to coleopteran insects and, in addition, both produce flat rectangular crystal inclusions and have a major polypeptide component of 64-68 kDa. Specifically, the var. san diego shows toxicity to:

| Order Activity | Species | Common name | |
|---|---|---|---|
| Coleoptera | Pyrrhalta luteola<br>Leptinotarsa decemlineata<br>Haltica tombacina<br>Anthonomus grandis<br>Otiohynchus sulcatus<br>Tenebrio molitor<br>Diabrotica undecimpunctata | Elm leaf beetle<br>Colorado potato beetle<br>-<br>Boll weevil<br>Black vine weevil<br>Yellow mealworm<br>Western spotted cucumber beetle | + + + +<br>+ + +<br>+ + +<br>+ + +<br>+ +<br>+ +<br>+ |
| but is not pathogenic to : | | (different scale) | |
| Coleoptera<br><br><br>Diptera<br>Lepidoptera | Attagenus unicolor<br>Gibbium psylloides<br>Tribolium castaneum<br>Aedes aegypti<br>Spodoptera exigua<br>Trichoplusia ni | Black carpet beetle<br>-<br>Red flour beetle<br>Yellow fever mosquito<br>Beet armyworm<br>Cabbage looper | -<br>-<br>-<br>-<br>-<br>- |
| The var. tenebrionis exhibits toxic activity to : | | | |
| Coleoptera | Leptinotarsa decemlineata<br>Diabrotica virgifera virgifera<br>Diabrotica undecimpunctata | Colorado potato beetle<br>Western corn rootworm<br>Southern corn rootworm | + + + +<br>+ +<br>+ |

In order to demonstrate that the insecticidal activity of B. thuringiensis var. san diego was due to the major protein having a molecular weight of 64 kDa, the 64 kDa protein was isolated, purified by HPLC and tested against elm leaf beetle (P. luteola) in a quantitative bioassay. A plot of log dose vs. probit mortality gave an $LC_{50}$ of 0.12 μg toxin protein per square centimeter of leaf surface.

The number, shape and composition of the inclusions vary for different subspecies of B. thuringiensis. The most prevalent crystal type inclusion (as exemplified by var. kurstaki) shows toxicity to lepidopteran larvae, exists as one (but occasionally 2 or 3) inclusion per cell, has a bipyramidal shape (although irregular shapes have been found) and is comprised of the δ-endotoxin or P1 polypeptides having a molecular weight of 130-140 kDa. This protoxin is subsequently digested to the active P1 toxin of approximately 55-70 kDa. In the case of B. thuringiensis var. israelensis, the major polypeptide in polyacrylamide gels, under native or denaturing conditions, is about 28 kDa, although larger than 65 kDa species have recently been reported (Lee et al. Biochem. Biophys. Res. Commun. 126:953-960). The most potent toxic protein of israelensis is about 130 kDa in molecular weight (Ward, E.S. et al. (1987) Nucl. Acids Res. 15:7195). In the kurstaki subspecies both P1 and P2 appear to be protoxins and, while the lepidopteran-specific P1 or δ-endotoxin has a molecular weight of approximately 135 kDa, the molecular weight of the P2 component, active against Lepidoptera and Diptera, is approximately 65 kDa (Yamamoto et al. (1981) Biochem. Biophys. Res. Commun. 103:414-421). In the two B. thuringiensis subspecies found to be pathogenic to Coleoptera (namely, tenebrionis and san diego) the major peptide component was found to have a molecular weight of 64-68 kDa.

The different-toxins isolated from various subspecies have been further differentiated on the basis of different amino acid composition and lack of immunological homology. Antiserum against kurstaki P1 (δ-endotoxin) protein (toxic to Lepidoptera) did not cross react with protein of israelensis (active against dipteran but not lepidopteran larvae). Similarly, antiserum against san diego (pathogenic to Coleoptera but not to Lepidoptera nor Diptera) did not cross react with the Lepidopteran-specific toxin of var. kurstaki. strains HD-1 and HD-73.

The insecticidal activity in the Lepidopteran active var. kurstaki strains resides in a protoxin (with a molecular weight of approximately 135 kDa) which is converted by insect gut proteases to the active toxin molecule having a molecular weight of approximately 65 kDa. The active portion of the protoxin is located in the aminoterminal half of the polypeptide. A series of deleted derivatives of the cloned gene of the kurstaki HD1 (Dipel) protoxin were used to localize the active domain of the gene. It was shown that the amino terminal 55% of the protoxin protein is sufficient for lepidopteran toxicity. Deletions to the 50th codon from the 5′ end or to the 603rd codon from the 3′ end abolish toxicity, while deletions to the l0th codon from the 5′ end or to the 645th codon from the 3′ end do not. The 3′ end of the crystal protein gene from codons 645 to 1176 is not essential for toxicity, and the first 10 codons can be replaced by two foreign segments (H. Schnepf et al. (1985) J. Biol. Chem. 260:6273-6280; M. Adang et al. (1985) Gene 36:289-300). The function of the carboxyl half of the protoxin molecule is not yet known, although there appears to be considerable conservation at the amino acid level based on comparison of nucleotide regions of four B. thuringiensis genes (i.e., var. kurstaki HD1 (Dipel), sotto, kurstaki HD73 and kurstaki HD1). The carboxyl terminal may function in determining specificity of the protoxin molecule, in determining the attachment of toxin to receptor sites or in determining the deposition of the protoxin as an inclusion. Cloned genes and subclones will be useful in further elucidating the functions of various regions of the toxin molecule with the aim of producing novel genes encoding proteins with enhanced toxicity or altered host specificity. A relationship of toxin activity to a particular part of the protein has not been reported for toxins active against coleopterans.

Toxin genes from several subspecies of B. thuringiensis (for example, var. kurstaki HD1, var. kurstaki HD1(Dipel), kurstaki HD73, berliner 1715, thuringiensis HD2, sotto, aizawai, subtoxicus, israelensis), have been cloned and the recombinant clones of var. kurstaki HD1 and israelensis were found to be toxic to insect larvae. These clones produced toxin in E. coli, if the DNA fragment was derived from a plasmid. The chromosomal gene from either var. berliner or var. kurstaki, however, did not reproducibly produce toxin detectable by immunological procedures or bioassay in E. coli. It has been shown for B. thuringiensis var. kurstaki that the chromosomally encoded parasporal protein is synthesized later in sporulation than the plasmid encoded protein, so gene location may reflect different modes of regulation (A. Aronson et al. (1986) Microbiol. Rev. 50:1-24).

A method has been disclosed (C.A. Stock, EPO Patent Application Publication No. 0 255 311) for introducing the structural gene from B. thuringiensis var. kurstaki HD73 or var. kurstaki HD1 (Dipel) into P. cepacia which colonizes roots or leaves of plants. Also, truncated crystal protein genes introduced into DNA plasmids have been used (M.J. Adang, EPO Patent Application Publication No. 0 209 370) to produce protein fragments which are precursors of toxins. More recently, modified genes from B. thuringiensis var. berliner 1715 were inserted into tobacco plants and sufficient toxin was produced to protect the transformed tobacco plants against tobacco hornworm larvae (Vaeck et al. (1987) Nature 328:33-37; M.J. Adang et al. (1985) Gene 36:289-300). The bacterial genes were stably integrated within the tobacco plants and were inherited by subsequent generations.

With respect to coleopteran active toxin genes, Herrnstedt et al. (1986), supra, disclosed that a 5.8 kb BamHI fragment of B. thuringiensis var. san diego DNA was cloned into E. coli using the pBR322 cloning vector. The protein expressed was toxic to P. luteola (Elm leaf beetle) and had a molecular weight of approximately 83 kDa. This 83 kDa toxin product from the B. thuringiensis var. san diego gene is larger than the 64 kDa crystal toxin isolated from B. thuringiensis var.san diego cells, suggesting that the B. thuringiensis var. san diego crystal protein may be synthesized as a larger precursor molecule that is processed by B. thuringiensis var. san diego but not by E. coli prior to being formed into a crystal.

Of five cloned and sequenced δ-endotoxin genes from different B. thuringiensis subspecies, four were lepidopteran active and plasmid encoded (i.e., var. kurstaki HD1 (Dipel), kurstaki HD73, sotto and kurstaki HD1) and had virtually identical sequences from several hundred nucleotides 5′ to the coding region to about 900 bp within the coding region. The fifth cloned gene, from the lepidopteran active var. berliner 1715 and derived from a chromosomal gene, showed almost no homology to the others. The upstream region of one of the three, virtually identical δ-endotoxin genes was found to have three different promoter sites: one recognized by E. coli and two by B. thuringiensis RNA polymerases. Transcripts mapping to the downstream Bacillus promoter were found at about stage II of sporulation, but decreased at about stage III or IV, at which time transcripts from the upstream promoter appeared. The upstream region of the δ-endotoxin gene also embodied two regions of hyphenated dyad symmetry. These were located such that they include both transcription start sites and therefore could be sites where regulatory elements bind. In addition, a potential ribosome binding site is situated three bases upstream from the initiator ATG codon.

A preliminary description of the coleopteran-active toxin gene located within a 5.8 kb fragment from B. thuringiensis var. san diego (B.t.sd) by Herrnstadt et al. (1986) supra, did not fully disclose the toxin gene; i.e., the nucleotide sequence of the DNA fragment, restriction maps of the fragment and amino acid sequence of the toxin protein were not provided in the publication (Herrnstadt et al. (1986), supra). Whether the DNA fragment containing the gene for the B.t.sd toxin was derived from chromosomal or plasmid DNA was not determined by the above authors. Also, public availability of this organism was not disclosed in the report.

Transcription studies with a limited number of genes have shown that homologous δ-endotoxin genes can give rise to polypeptide toxins that may have different biological activity profiles. For example, the kurstaki HD1 and HD73 genes are virtually identical, yet the resultant toxins have different 50% lethal concentrations for certain Lepidoptera (Yamamoto et al. (1983) Arch. Biochem. Biophys. 227:233-241). Moreover, the plasmids containing these protoxin genes, when transferred to B. cereus, are regulated differently (Minnich et al. (1984) J. Bacteriol. 158:447-454). It is believed that factors other than promoter sequences and unique forms of RNA polymerase are involved in regulation.

Most curious was the report by Vaeck et al. (1987), supra, that in transgenic tobacco plants harboring either the intact bt2 gene (1155 codons of the toxin gene from var. berliner 1715) or a truncated gene derivative (860 codons of the 5′ region of the toxin gene), only the truncated bt2 genes gave rise to expression levels that were strongly insecticidal. No significant insecticidal activity was obtained using the intact bt2 coding sequence, despite the fact that the same promoter was used to direct its expression. Intact Bt2 protein and RNA amounts in the transgenic plant leaves were 10-50 times lower than those for the truncated B. thuringiensis polypeptides. The reason why the complete bt2 gene is not expressed at an equally high level in plant cells is not known.

In general, B. thuringiensis subspecies contain a substantial portion of their potential genetic information in plasmids. On the basis of cross-hybridization, protoxin genes were found primarily in large ( >30 MDa ) plasmids and, in some cases, on more than one plasmid in a given subspecies (Aronson et al. (1986) Microbiol. Rev. 50:1-24). In the subspecies kurstaki HD1, plasmids of 110, 29 and 4.9 MDa have functions in regulating protoxin synthesis. Some of these smaller plasmids that have a regulatory role do not contain protoxin genes. In other subspecies (for example, var. kurstaki HD73), a simpler regulatory profile exists in that regulatory genes have been incorporated into the same plasmid that carries the protoxin gene.

Transfer of plasmids via cell mating of B. thuringiensis was first reported by Gonzales et al. (1982) (Proc. Natl. Acad. Sci. 79:6951-6955). Subsequently, Klier et al. (1983) (Mol. Gen. Genet. 191:257-262) reported the transfer of cloned protoxin genes from B. subtilis to B. thuringiensis by cell mating. The recombinant plasmid containing the plasmid encoded berliner 1715 δ-endotoxin gene was transferred from B. subtilis into var. kurstaki D1 as well as into var. israelensis. In the latter case, the lepidopteran active berliner 1715 and dipteran active israelensis were both expressed in normal amounts, indicating that two different toxins could be expressed in the same strain (possibly broadening the host range of strains used in biological control).

## SUMMARY OF THE INVENTION

It is the overall object of the present invention to provide a means for plant protection against insect damage.

An object of this invention is to provide a cloned gene coding for a coleopteran-insecticidal protein of B. thuringiensis var. tenebrionis.

Another object of this invention is to provide a modified cloned gene of B. thuringiensis var. tenebrionis coding for a modified protein having coleopteran-insecticidal activity.

Another object of this invention is to provide a novel 73 kDa protein having coleopteran-insecticidal activity.

It is a particular object of this invention to provide a biocontrol agent toxic specifically to coleopteran insects.

This invention provides a biocontrol method for plant protection against, in particular, Colorado potato beetle, Western corn rootworm and Southern corn rootworm (examples of coleopteran insects).

This invention provides a method for the production of the insecticidal δ-endotoxin protein in genetically altered bacteria.

In particular, this invention provides a method for the production of δ-endotoxin protein as a product of the δ-endotoxin gene from B. thuringiensis var. tenebrionis expressed in transformed E. coli or other gram negative cells. The resultant biologically active product exhibits a specific activity profile showing toxicity to coleopteran insects.

Another object of this invention is to provide a method for the introduction of a gene encoding a crystal protein of a strain of B. thuringiensis into E. coli or other gram negative strains and transforming the agriculturally benign bacterial strain into one toxic to insects.

This invention is exemplified by demonstrating expression of the δ-endotoxin gene of B. thuringiensis var. tenebrionis in a gram negative host (E. coli). Such genetically transformed bacterial strains provide bioregulatory methods of plant protection and agricultural pest control.

In addition, this invention provides a method for modifying the δ-endotoxin gene prior to its utilization in a recombinant construct for expression in transformed bacterial systems of crystal protein toxin having preserved insecticidal activity.

Specifically, in this invention the δ-endotoxin gene from B. thuringiensis var. tenebrionis encoding the crystal toxin was modified by specific deletion in the N-terminal area of the coding region. Expression of this gene derivative resulted in the production of protein toxin having coleopteran-specific activity.

This invention also provides a method for the temporal regulation of the insecticidal crystal protein gene in B. thuringiensis var. tenebrionis.

In addition, this invention provides a method for a B. thuringiensis var. tenebrionis promoter-induced regulation of expression of a B. thuringiensis crystal protein gene and production of the corresponding antigen having insecticidal activity.

## BRIEF DESCRIPTION OF THE FIGURES

Figure 1 is a comparison of partial restriction endonuclease maps of DNA fragments harboring the δ-endotoxin genes of B. thuringiensis var. tenebrionis in (A) pNSBP544 (described in example 1) (B) p544HindSC (described in example 2) and (C) p544Pst-Met5 (described in example 2). (D) The linker built to restore the Met codon in the B. thuringiensis var. tenebrionis sequence prior to the Pst1 site. Bold arrow shows direction of the crystal protein transcription. Shaded areas indicate crystal protein coding DNA. Thick lines indicate vector.

Figure 2 is the DNA sequence of the crystal toxin gene from B. thuringiensis var. tenebrionis. Nucleotide sequence of 2400 bp of var. tenebrionis DNA encoding the 73 kDa crystal protein. The amino acid sequence for the open reading frame is given below. The putative Shine-Dalgarno sequence is boxed. Wavy arrows show the position of transcription initiation in B. thuringiensis (Bt) and E. coli (Ec) as determined by Mung bean nuclease mapping. An inverted repeat (IR) beyond the 3′ end of the gene is shown by arrows.

Figure 3 is an immunoblot analysis of the protein synthesized in the E. coli transformants. (A) Transformants synthesizing the 73 kDa and 65kDa polypeptide components. Lane 1, 250 ng var. tenebrionis crystal protein. Lane 2, 50 ng crystal protein. Lane 3, 25 µg protein from sporulating (stage III-IV) cells of var. tenebrionis. 50 µg protein from each of the following samples were analyzed in lanes 4-7. Lane 4, E. coli pIC20H. Lane 5, E. coli pNSBP544. Lane 6, E. coli 544Bam(-)SC. Lane 7, E. coli 544HindSC. Protein standards with their size (kDa) are shown on the left. (B) Transformants synthesizing only the 65kDa component. Lane 1, pIC20H vector control. Lane 2, pNSBP544 containing the complete B. thuringiensis var. tenebrionis gene. Lane 3, p544PstSC. Lane 4, p544Pst4 without a BamHI site. Lane 5, p544Pst-Met5. Lane 6, P544Pst13 without a BamHI site.

Figure 4 is a Southern blot analysis. Plasmid DNA samples of several strains of B. thuringiensis and E. coli recombinant strain NSBP544 were separated on a 0.6% agarose gel (panel A) and probed with $^{32}P$-labelled 0.7 kb internal EcoRI fragment (panel B). Lanes 1-5 contained the following B. thuringiensis strains: (1) thuringiensis HD2, (2) kurstaki HD1, (3) kurstaki HD73, (4) israelensis HD567 and (5) tenebrionis. Individual components of the plasmid array of var. tenebrionis are pointed by dots and the size (in MDa) of the presumptive crystal coding plasmid is shown on the right margin. Panel C. Purified total DNA (lanes 1-3) and plasmid DNA (lanes 4-6) preparations obtained from var. tenebrionis were digested with BamHI (lanes 1,4), HindIII (lanes 2,5) and EcoRI (lanes 3,6) and screened with the same probe as above. The relative mobilities and the size (in kb) of the linear Mr markers are shown on the left. The sizes (in kb) of the fragments exhibiting specific hybridization are shown on the right.

Figure 5 aligns and compares the hydropathic profiles of B. thuringiensis var. tenebrionis and var. kurstaki HD73 crystal protein genes. (A) Graphic alignment of var. tenebrionis over var. kurstaki HD73 amino acid sequence. Numbering is from the N-terminal methionine. Conserved residues are indicated by vertical bars. Inserted gaps are represented by horizontal bars. Hydropathic profiles of the var. tenebrionis (B) and var. kurstaki HD73 (C) proteins were calculated according to Kyle and Doolittle (1982, J. Mol. Biol. 157:105-132) using a window of 7 amino acids. For optimal alignment, hydropathic profiles include the gaps shown in (A).

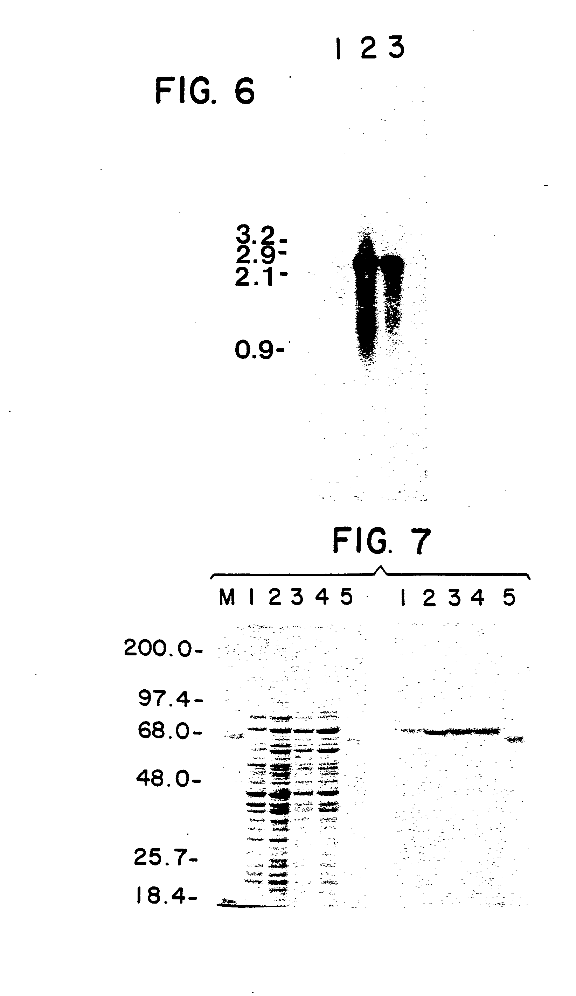

Figure 6 demonstrates the time of appearance of the crystal protein transcript. Total RNA (5 µg each) extracted at mid-log (lane 1), early stationary or $t_2$ (lane 2) and mid-stationary or $t_7$ (lane 3) growth phases were separated on a 1.0% agarose gel. Crystal protein specific transcripts were identified by using a 0.7 kb internal EcoRI fragment of the crystal protein gene of B. thuringiensis var. tenebrionis. The sizes (in kb) of marker Brome mosaic virus RNA transcripts are shown in the left margin.

Figure 7 documents the time of appearance of the crystal protein antigen. Total protein (25 µg each) extracted from various growth stages were separated on a 0.1% SDS-10% polyacrylamide gel. Crystal specific antigen in the mixture was identified by immunoblot analysis. Coomassie blue stained gel is shown on panel A and the corresponding immunoblot on panel B. Protein samples obtained from mid-log (lane 1), early stationary, $t_0$, at the onset of stationary phase (lane 2), early stationary, $t_2$, (lane 3) and mid-stationary, $t_7$, (lane 4) cells were analyzed. Lane 5 contains 50 ng of purified crystal protein preparation. Lane M of panel A contains protein molecular weight standards. The sizes (in kDa) of the markers are shown on the left margin. The position of the minor 73 kDa crystal component (in panel B) is indicated by an arrow.

## DETAILED DESCRIPTION OF THE INVENTION

The following definitions are provided in order to provide clarity as to the intent or scope of their usage in the specification and claims.

Expression refers to the transcription and/or translation of a structural gene.

Promoter refers to the nucleotide sequence(s) at the 5′ end of a structural gene which direct(s) the initiation of transcription. Promoter sequences are necessary, but not always sufficient, to drive the expression of a downstream gene. In prokaryotes, the promoter drives transcription by providing binding sites to RNA polymerases and other initiation and activation factors. Usually promoters drive transcription preferentially in the downstream direction, although promotional activity can be demonstrated (at a reduced level of expression) when the gene is placed upstream of the promoter. The level of transcription is regulated by promoter sequences. Thus, in the construction of heterologous promoter/structural gene combinations, the structural gene is placed under the regulatory control of a promoter such that the expression of the gene is controlled by promoter sequences. The promoter is positioned preferentially upstream to the structural gene and at a distance from the transcription start site that approximates the distance between the promoter and the gene it controls in its natural setting. As is known in the art, some variation in this distance can be tolerated without loss of promoter function.

A gene refers to the entire DNA portion involved in the synthesis of a protein. A gene embodies the structural or coding portion which begins at the 5′ end from the translational start codon (usually ATG) and extends to the stop (TAG, TGA or TAA) codon at the 3′ end. It also contains a promoter region, usually located 5′ or upstream to the structural gene, which initiates and regulates the expression of a structural gene.

Structural gene is that portion of a gene comprising a DNA segment encoding a protein, polypeptide or a portion thereof, and excluding the 5′ sequence which drives the initiation of transcription. The structural gene may be one which is normally found in the cell or one which is not normally found in the cellular location wherein it is introduced, in which case it is termed a heterologous gene. A heterologous gene may be derived in whole or in part from any source known to the art, including a bacterial genome or episome, eukaryotic, nuclear or plasmid DNA, cDNA, viral DNA or chemically synthesized DNA. A structural gene may contain one or more modifications in either the coding or the untranslated regions which could affect the biological activity or the chemical structure of the expression product, the rate of expression or the manner of expression control. Such modifications include, but are not limited to, mutations, insertions, deletions and substitutions of one or more nucleotides. The structural gene may constitute an uninterrupted coding sequence or it may include one or more introns, bounded by the appropriate splice junctions. The structural gene may be a composite of segments derived from a plurality of sources, naturally occurring or synthetic. The structural gene may also encode a fusion protein.

Plant tissue includes differentiated and undifferentiated tissues of plants, including but not limited to, roots, shoots, leaves, pollen, seeds, tumor tissue and various forms of cells in culture, such as single cells, protoplasts, embryos and callus tissue. The plant tissue may be in planta or in organ, tissue or cell culture.

Homology refers to identity or near identity of nucleotide or amino acid sequences. As is understood in the art, nucleotide mismatches can occur at the third or wobble base in the codon without causing amino acid substitutions in the final polypeptide sequence. Also, minor nucleotide modifications (e.g., substitutions, insertions or deletions) in certain regions of the gene sequence can be tolerated and considered insignificant whenever such modifications result in changes in amino acid sequence that do not alter functionality of the final product. It has been shown that chemically synthesized copies of whole, or parts of, gene sequences can replace the corresponding regions in the natural gene without loss of gene function. Homologs of specific DNA sequences may be identified by those skilled in the art using the test of cross-hybridization of nucleic acids under conditions of stringency as is well understood in the art (as described in Hames and Higgens (eds.) (1985) Nucleic Acid Hybridization, IRL Press, Oxford, UK). Extent of homology is often measured in terms of percentage of identity between the sequences compared. Thus, in this disclosure it will be understood that minor sequence variation can exist within homologous sequences and that any sequences exhibiting at least 75% homology are deemed equivalent.

Derived from is used to mean taken, obtained, received, traced, replicated or descended from a source (chemical and/or biological). A derivative may be produced by chemical or biological manipulation (including but not limited to substitution, addition, insertion, deletion, extraction, isolation, mutation and replication) of the original source.

Chemically synthesized, as related to a sequence of DNA, means that the component nucleotides were assembled in vitro. Manual chemical synthesis of DNA may be accomplished using well established procedures (M. Caruthers (1983) in Methodology of DNA and RNA Sequencing, Weissman (ed.), Praeger Publishers (New York), Chapter 1), or automated chemical synthesis can be performed using one of a number of commercially available machines.

Crystal protein or crystal toxin or δ-endotoxin refers to the major protein component of the parasporal crystals formed in strains of B. thuringiensis. This protein component exhibits selective pathogenicity to different species of insects. The molecular size of the major protein isolated from parasporal crystals varies depending on the strain of B. thuringiensis from which it is derived. Crystal proteins having molecular weights of approximately 132, 65, and 28 kDa have been reported. B. thuringiensis strains that are toxic to Lepidoptera contain the 135 kDa polypeptide, those that are coleopteran active have a toxin protein of approximately 65 kDa, whereas B. thuringiensis strains specific for Diptera produce a 28 kDa polypeptide toxin and a 130 kDa toxin, the latter having the most Dipteran toxicity. It has been shown that in B. thuringiensis var. kurstaki the approximately 135 kDa polypeptide is a protoxin that is cleaved in the insect midgut to form an approximately 68 kDa toxin. It was further elucidated that the active portion of the protoxin is located in the N-terminal half of the polypeptide. It is the N-terminal portion of the polypeptide that is retained after cleavage to form the toxin.

The crystal protein gene or δ-endotoxin gene refers to the DNA sequence encoding the insecticidal crystal protein in either full length protoxin or toxin form, depending on the strain of B. thuringiensis from which the gene is derived.

A recombinant DNA molecule refers to constructions in which DNA sequences which normally do not coexist are artificially brought together through the use of cleavage and ligation enzyme techniques of genetic engineering.

Cloning refers to a procedure used in genetic engineering to relocate a segment of DNA from its natural location to a different site where it will be reproduced.

The cloning process involves excision and isolation of the desired DNA segment, insertion of the piece of DNA into a vector molecule and incorporation of the recombinant vector into a cell where multiple copies or clones of the DNA segment will be replicated.

The present invention is based on the report that, contrary to the activity of most strain of B. thuringiensis which are toxic to Lepidoptera and some B. thuringiensis strains which act against Diptera, B. thuringiensis var. tenebrionis exhibits specificity to Coleoptera (A. Krieg et al. (1983), supra). A second B. thuringiensis strain, i.e., san diego, was also found to be coleopteran toxic. Several agronomically important insect pests belong to the Order Coleoptera (for example, Colorado potato beetle, boll weevil and corn rootworm). Thus, the host range was expanded for B. thuringiensis, a pathogen highly favored for use commercially as an agricultural insecticide. In addition, this report opened up the possibility of inventing (providing) a novel means for controlling several economically important pests, including the boll weevil (Anthonomus grandis), the Colorado potato beetle (Leptinotarsa decemlineata) and, in particular, the western corn rootworm (Diabriotica virgifera virgifera).

In the present invention the gene coding for the crystal protein of B. thuringiensis var. tenebrionis is utilized to provide a means of biological control of insect pests for the protection of plants. E. coli transformed with the B. thuringiensis var. tenebrionis δ-endotoxin gene are rendered toxic to coleopteran insects. Methods and compositions are provided for controlled expression of a gene in a bacterial host (described in example 3). DNA sequences (example 4) which comprise the structural gene and its promoter region are employed. These include the RNA polymerase recognition and binding sites and the transcriptional initiation sequence (cap site). The regulatory system is found on a DNA fragment of fewer than 600 bp located on the 5′ flanking region of the δ-endotoxin gene upstream of the translational initiation codon. As used herein, the term upstream means in the direction opposite to transcription, while the term downstream means in the direction of transcription.

The promoter and structural gene sequences may be combined with a prokaryotic replication system (which allows amplification in a prokaryotic host), markers for selection and other DNA regions. The vector can also include other DNA sequences having restriction site(s) for the insertion of genes under transcriptional control of the promoter to provide a vector for the regulated transcription and translation of the inserted genes in said bacterial host.

The δ-endotoxin gene can be isolated from the 90 kDa plasmid of var. tenebrionis as a 5.9 kb BamHI fragment (Figure 4). The entire fragment may be used by introduction into a BamHI insertion site on an appropriate vector. The 5.9 kb BamHI insert may be oriented in either the same or in the opposite direction with respect to a preexisting lacZ promoter in the vector. When the orientation of the gene is the same as that of the lacZ promoter, transcription is augmented several fold. The recombinant plasmid vector may be used to transform an appropriate host cell. The inserted gene, under control of its own regulatory system and translation initiation factors, will then be expressed as a native polypeptide corresponding to the inserted gene.

Alternatively, the 5.9 kb DNA fragment can be further restricted. The 5′ and 3′ regions flanking the structural gene can be subjected to controlled digestion using appropriate restriction endonucleases or using a double stranded exonuclease for a period of time chosen to remove a desired number of base pairs. The resulting shortened fragment can then be inserted into a vector using existing or attached restriction sites compatible with the insertion sites of the vector. In this way, the regulatory regions as well as the coding regions may be identified and modified.

In an exemplary embodiment of the present invention as described in example 2, a B. thuringiensis var. tenebrionis gene encoding toxin protein was introduced into E. coli as one of three DNA fragments: as a 5.9 BamHI fragment, as a 3.0 kb HindIII subclone or as a deletion derivative, p544Pst-Met5, enzymatically derived from the 5.9 BamHI fragment. Specifically, the gene fragment encoding the δ-endotoxin of B. thuringiensis var. tenebrionis was introduced into E. coli by cloning the toxin protein encoding sequences into the pUC12 plasmid and using this recombinant plasmid to transform E. coli. All three gene fragments were expressed; toxic, 73 kDa polypeptides were produced in expression systems utilizing the 5.9 BamHI fragment or the 3.0 kDa HindIII subclone, whereas a 65kDa polypeptide toxin was obtained with the N-terminal deletion derivative, p544Pst-Met5. The 65 kDa recombinant polypeptide expression product differs from the 65 kDa polypeptide isolated as a crystal toxin in two reports. The natural 65 kDa isolates contain a mixture of closely similar proteins having variability at the N-terminus, whereas the recombinant expression product is presumably uniform and has an N-terminal methionine. (Several insecticidal polypeptides having a molecular weight of approximately 65 kDa may be isolated from B. thuringiensis var. tenebrionis and, presumably, each represents a different extent of proteolytic processing of the larger 73 kDa crystal protein. Although an active insecticidal crystal protein can be produced from a B. thuringiensis var. tenebrionis crystal toxin gene modified (deletions) in the N-terminal region, deletions greater than four or five amino acids at the C-terminus could not be tolerated and resulted in a greater than ten fold loss in insecticidal activity.

It is desirable to allow for amplification of the vector in a bacterial host. This allows large quantities of the vector to be grown in well characterized bacterial systems. Suitable prokaryotic replication systems are well known to those of ordinary skill in the art and include plasmids such as pBR322, pRK290, ColE1 and bacteriophages. The prokaryotic replication system will be understood to include an origin of replication recognizable by a prokaryotic host, and will usually include one or more markers for the selection of transformants in the prokaryotic host. Such markers include, i.e., biocide resistance and toxin resistance.

In the exemplified method for preparing the vectors of the invention, the δ-endotoxin gene is inserted into a suitable prokaryotic plasmid where it is amplified. It may be desirable to select a replication system capable of replicating in a eukaryotic host. Suitable replication systems may be derived from various viral systems, especially those which are able to exist in specific hosts at high copy numbers. In these situations, the δ-endotoxin gene would be inserted directly into a eukaryotic (plant) host for expression of the toxin protein at a suitable cellular location. Standard technology used to transform plants and commonly employed by those skilled in the art are detailed in Adang and Kemp, EPO publication No. 0 142 924 Published 05/29/85; Vaeck et al. (1987), supra).

The insect toxin present within the bacterial carrier system is derived from B. thuringiensis. Various strains of B. thuringiensis exhibit different toxicity spectra to insect pests. For example, B. thuringiensis var. kurstaki is toxic to Lepidoptera, the var. israelensis is active against Diptera whereas, the subspecies tenebrionis and san diego act against Coleoptera. Some strains of B. thuringiensis var. kurstaki are toxic to both Lepidoptera and Diptera; however, in this case, different proteins are implicated in conferring such broad spectrum toxicity.

The methods of the present invention can utilize any of the toxin genes of B. thuringiensis. The toxin gene of B. thuringiensis var. tenebrionis was chosen to obtain the desired toxicity spectrum for the resultant genetically modified bacterium. To further alter or broaden the target range, it may be desirable to introduce more than one toxin gene into the recombinant bacterial system. Two or more toxin genes having different insect specificities can be inserted in tandem arrangement or can be introduced on individual plasmids. Part of the existing B. thuringiensis var. tenebrionis δ-endotoxin gene may have to be altered in order to allow insertion of an additional structural gene of interest. This may be accomplished by cleaving the gene at an internal restriction site and treating the resulting linearized DNA sequences with a double stranded exonuclease to remove a desired portion of the B. thuringiensis var. tenebrionis structural gene and allow insertion of the desired structural gene. The structural gene can be inserted in the proper orientation for expression by employing different linkers at the ends of the gene. Disclosure of the sequences of our invention makes possible the necessary modifications and refinements of the toxin gene to enable these extended goals.

Although E. coli cells are most frequently used to show expression of a recombinant gene construct, other gram negative cells, instead, may be used to show specific DNA expression. For example, a B. thuringiensis crystal protein gene has been introduced and expressed in Rhizobium (Adang et al., EPO Patent Application Publication No, 0 209 370) and in Pseudomonas (Stock et al., EPO Application Publication No. 0 255 311). As described in the examples of our invention recombinant plasmids containing the toxin protein sequences were used to transform E. coli cells. Two recombinant constructs, pNSBP544 and p544HindSC, produced 73 kDa and 65 kDa polypeptides whereas the recombinant p544Pst-Met5 derivative produced almost exclusively the 65 kDa polypeptide. The expressed protein (the 73 kDa as well as the 65 kDa polypeptide species) cross-reacted with an antiserum against the purified 65·kDa crystal protein of var. tenebrionis (Figure 3). Crystals isolated from var. tenebrionis contain both the 73 and 65 kDa components, However, sporulating var. tenebrionis cells contain predominantly an immunoreactive 73 kDa peptide that corresponds to the larger of the products of the recombinant constructions. It is believed that the larger translation product is converted into the lower molecular weight peptide through proteolytic processing (K. Bernhard (1986) FEMS Microbiol. Lett. 33.:261-265).

In the present invention the time of appearance of the insecticidal crystal protein transcript and the corresponding crystal protein antigen of B. thuringiensis var. tenebrionis was determined as described. In general, the expression of the crystal protein genes of all B. thuringiensis varieties have been shown to start only during mid-sporulation stages. The temporal regulation of expression of the crystal protein genes is well reflected by their highly conserved promoter regions that are recognized by sporulation specific forms of RNA polymerase (E. Ward et al. (1986) J. Mol. Biol. 191:1-11; H. Wong et al. (1983) J. Biol. Chem. 258:1960-1967). Comparison of the nucleotide sequence of the crystal protein genes of B. thuringiensis var. tenebrionis and other B. thuringiensis varieties reveals that apart from differences in structural genes, their promoter regions share only a slight homology.

It is shown in the present invention that in B. thuringiensis var. tenebrionis, in contrast to other varieties of B. thuringiensis, the crystal protein transcript and corresponding antigen are made in detectible levels in vegetative cells (described in example 6). The expression of the crystal protein gene increases dramatically and reaches a plateau in early stationary cells. In addition, results from immunoblotting analysis suggest that the crystal protein antigen is made in vegetative cells as a 73 kDa peptide which by proteolytic processing is converted to a 65 kDa peptide to some extent in stationary cells.

The temporal control of expression of the crystal protein gene exhibited by the promoter region of B. thuringiensis var. tenebrionis may be utilized advantageously in modified systems to regulate production of specific insecticidal antigen. For example, the promoter region of the B. thuringiensis var. tenebrionis crystal protein gene can be fused to toxin genes from other B. thuringiensis varieties. It is also feasible to put other foreign structural genes of interest under the control of the B. thuringiensis var. tenebrionis insecticidal crystal protein promoter in order to achieve regulation of expression similar to that observed for the crystal toxin in B. thuringiensis var. tenebrionis The technology needed for such genetic engineering is standard and commonly employed by those skilled in the art.

In the present invention, the product of expression-a toxin - has a high potential for use in the management of insect pest populations. Purified recombinant products may be conveniently applied onto the leaves of appropriate host plants by spraying. In addition, the toxin product may be supplemented with chemical or fungicidal additives in an effort to expand the spectrum of target pests, to extend the duration of effectiveness of the applied toxin or to help stabilize the formulation of the toxin. The bacterium selected to act as carrier for delivering an insect toxic protein to the surface of plants may, in principle, be chosen from a wide range of bacteria that colonize diverse plants but are not pathogenic to plants.

In this invention, genetically altered E. coli carrying the δ-endotoxin gene from B. thuringiensis var. tenebrionis were found to be toxic to insects as measured with insect diet bioassays. Total protein was extracted from recombinant clones and an aliquot from each clone was spread onto individual potato leaves or applied to germinating corn kernels and fed to neonate larvae of Colorado potato beetle (Leptinotarsa decemlineata), western corn rootworm (Diabriotica virgifera virgifera) or southern corn rootworm (Diabrotica undecimpunctata). Each clone was tested in duplicate and insect mortality was recorded.

It has been shown that recombinant vectors introduced into bacteria are not always stably maintained and, after a number of generations, the insect toxic phenotype may be lost. There are methods available to those skilled in the art to achieve stabilization. Often, a gene necessary for cell survival is incorporated into the recombinant vector carrying a desired gene, for example, the δ-endotoxin gene.

In order to colonize a genetically modified bacterial strain on plant leaves, plant leaves must be inoculated with an appropriate composition containing the desired bacterial strain. In general, inoculants can be applied at any time during plant growth. Inoculation of large fields of plant in particular can be accomplished most effectively by spraying.

The concentration of genetically transformed bacteria that will be necessary to produce sufficient toxin to act as an effective insecticide must be experimentally established in routine bioassays as described in Example 3. The effective insecticidal dosage will vary depending on the exact δ-endotoxin gene fragment introduced, the plasmid vector used, the bacterial strain transformed, the type of plant to be protected and the final formulation of the toxic composite.

Inoculating compositions must be suitable for agricultural use and dispersal in fields. Generally, components of the composition must be non-phytotoxic, non-bacteriostatic and non-bacteriocidal. Foliar applications must not damage or injure plant leaves. In addition to appropriate liquid or solid carriers, inoculating compositions may include sticking and adhesive agents, emulsifying and wetting agents, and bacterial nutrients or other agents to enhance growth or stabilize bacterial cells. Inoculating compositions for insect pest control may also include agents which stimulate insect feeding.

The feasibility of engineering plants to defend themselves against Lepidopteran insects which are sensitive to specific B. thuringiensis toxins (i.e., the Bt2 protein produced by the toxin gene from var. berliner 1715) has been demonstrated (Vaeck et al. (1987), supra). To protect plants fully against other lepidopteran insects which are less sensitive to the var. berliner 1715 toxin, higher levels of expression will be desirable and may be achieved using chimeric toxin genes containing stronger plant-specific promoters. For example the 35S promoter of cauliflower mosaic virus (Odell et al. (1985) Nature 313:810-812) directs a 10-50 fold higher expression than the regular T-DNA promoter in plants. In addition, it will be feasible to transform plants with chimeric toxin gene constructs having high specific activity against other important insect targets, in particular, the coleopteran pests.

Reviews describing methods of application of biological insect control agents and agricultural inoculation are available. See, for example, Couch and Ignoffo (1981) in Microbial Control of Pests and Plant Disease 1970-1980, Burges (ed.), Chapter 34, pp. 621-634; Corke and Rishbeth, ibid, Chapter 39, pp. 717-732; Brockwell (1980) in Methods for Evaluating Nitrogen Fixation, Bergersen (ed.) pp. 417-488; Burton (1982) in Biological Nitrogen Fixation Technology for Tropical Agriculture, Graham and Harris (eds.) pp. 105-114; and Roughly (1982) ibid, pp. 115-127.

Summarily, the present invention is a Coleopteran-specific toxin protein produced in recombinant systems containing the toxin gene from B. thuringiensis var. tenebrionis. This invention bears resemblance to the toxin produced by B. thuringiensis var. san diego. To date, there are only two isolates of B. thuringiensis that are known to be active against Coleoptera - namely, var. san diego and var. tenebrionis. Thus, a comparison of the properties of these two toxins (as both the native and recombinant proteins) is presented in order to provide a basis for the distinction and identity of the present invention.

TABLE 1

| | B . thuringiensis | |
|---|---|---|
| PROPERTY | tenebrionis | san diego |
| Date of discovery | 1983 (Krieg et al.) | 1986 (Herrnstadt et al.) |
| Bacterial source | Boehringer Mannheim Corp. | undisclosed |
| Toxin specificity | Coleoptera | Coleoptera |
| Form of crystal | Flat rectangle | Flat rectangle |
| Size of isolated crystal protein | 73 and 65 kDa | 64 kDa |
| $LC_{50}$ of purified crystal protein | 0.18 µg/$cm^2$ | 0.12 µg/$cm^2$ |
| Location of gene | 90 kDa plasmid | ? |
| Isolated gene fragment | 5.9 kb BamHI | 5.8 kb BamHI |
| subclone | 3.0 kb Hind111 | |
| subclone | p544Pst-met5 | |
| Cloning vector | pUC12 | pBR322 |
| Host cell | E. coli | E. coli |
| Gene sequence | disclosed in this invention | undisclosed |
| Restriction maps | disclosed in this invention | undisclosed |
| Amino acid sequence | disclosed in this invention | undisclosed |
| Promoter region | 600 bp (maximum) | ? |
| Oligonucleotide probe | 27-mer | 17-mer |
| Immunological cross-reactivity | not with : kurstaki HD73 | not with : kurstaki |
| HD73 | israelensis | kurstaki HD1 |
| Size of translation product | | |
| with BamHI fragment | 73 and 65 kDa | 83 kDa |
| with Hind111 fragment | 73 and 65 kDa | - |
| with 544Pst-met5 | 65 kDa | - |

The B. thuringiensis toxins isolated from both var. san diego and tenebrionis are similar in insect specificity, shape of toxin crystal and approximate size of the crystal toxin protein. Although both B. thuringiensis strains have 64-65 kDa protein species that exhibit Coleopteran toxicity, the var. tenebrionis has additionally a minor 73 kDa polypeptide species that is a toxin protein. In recombinant systems containing the toxin gene from var. tenebrionis, toxic 65 kDa and 73 kDa polypeptides are produced; these proteins are of the same size as those isolated from B. thuringiensis var. tenebrionis crystals. In contrast, recombinant systems containing the toxin gene from var. san diego produce an 83 kDa toxin which is larger than the 64 kDa polypeptide isolated from B. thuringiensis var. san diego crystals and, most probably, represents a precursor form of the toxin.

The following examples are offered by way of illustration and not by way of limitation.

The examples utilize many techniques well known and accessible to those skilled in the arts of molecular biology, the manipulation of recombinant DNA in bacteria and plant tissue. Except as noted hereafter, standard techniques for cloning, DNA isolation, amplification and purification, for enzymatic reactions involving DNA ligase, DNA polymerase, restriction endonucleases and the like, and various separation techniques are those known and commonly employed by those skilled in the art. A number of standard techniques are described in: Maniatis et al. (1982) in Molecular Cloning, Cold Spring Harbor Laboratory, New York; We (ed.) (1979) Meth. in Enzymol. 68; Wu et al. (eds.) (1983) Meth. Enzymol. 100 and 101; Grossman and Moldave (eds.) Meth. Enzymol. 65; Miller (ed.) (1972) Experiments in Molecular Genetics, Cold Spring Harbor, New York; Old and Primrose (1981) Principles of Gene Manipulation, University of California Press, Berkeley; Schleif and Wensink (1982) Practical Methods in Molecular Biology; Glover (ed.) (1985) DNA Cloning Vol. 1 and 11, IRL Press, Oxford, UK; Hames and Higgins (eds.) (1985) Nucleic Acid Hybridization, IRL Press, Oxford, UK; Setlow and Hollaender (1979)Genetic Engineering: Principles and Methods, Vols. 1-4, Plenum Press, New York, which are expressly incorporated by reference herein. Abbreviations and nomenclature are deemed standard in the field and commonly used in professional journals such as those cited herein.

The following strains have been deposited with the Northern Regional Research Laboratory, 1818 North University Street, Peoria, Illinois:

| Strain Accession No. | Date of Deposit | |
|---|---|---|
| E. coli MC1061/p544 HindIII | October 6, 1987 | B-18257 |
| E. coli MC1061/p544 Pst-Met5 | October 6, 1987 | B-18258 |

Example 1: Cloning of the δ-endotoxin gene of B. thuringiensis var. tenebrionis

B. thuringiensis var. tenebrionis was provided by Boehringer-Mannheim Corp., Mannheim, FRG, while other strains of B. thuringiensis were supplied by Dr. H. T. Dulmage, USDA, Brownsville, Texas. Total DNA from B. thuringiensis var. tenebrionis was isolated according to the method described by Kronstad et al. (1983) (J. Bacteriol. 154:419-428) and digested with BamHI. The BamHI fragments of B. thuringiensis var. tenebrionis total DNA were mixed with BamHI-digested, dephosphorylated pUC12 (C. Yannisch-Peron et al. (1985) Gene 33:103-119) and ligated in the presence of T-4 DNA ligase at 14° C for 18h. The ligated DNA mixture was introduced into competent E. coli JM83 cells. The transformation mixture was plated onto L-agar plates containing 50 µg/ml ampicillin and 30 µg/ml of the chromogenic substrate, 5-bromo-4-chloro-3-indoyl-β-D-galactoside. Recombinants were identified by the loss of β-galactosidase activity. Over 600 putative recombinant clones were obtained. Replica of the recombinant clones were made onto nitrocellulose membrane filters.

The nitrocellulose filters containing the lysates of putative recombinant clones were screened with a chemically synthesized 27-mer oligonucleotide probe. In order to make the probe, the N-terminal amino acid sequence for the δ-endotoxin protein of B. thuringiensis var. tenebrionis was obtained. First, crystal inclusions were purified from crude spore/crystal mixtures of B. thuringiensis var. tenebrionis by passing the mixture twice through linear (55-88%) sucrose gradients. Urea-denatured crystal protein sample (4.0 µg) was treated with trypsin and the tryptic fragments were separated on a Varian 5000 HPLC using a Vydac $C_8$ reverse-phase column with a linear gradient from 100% solvent A (0.1% trifluoroacetic acid, TFA, in water) to 40% solvent B (0.1% TFA in acetonitrile). Purified fragments obtained from clean peaks were subjected to N-terminal protein sequencing on a microsequenator. Next, a 27-mer oligonucleotide (TATAAACAATATCCATTTGTTATGATG) corresponding to the sequence of nine N-terminal amino acids of a tryptic fragment was prepared by oligonucleotide synthesis. The preferred codon usage in the crystal protein genes of B. thuringiensis var. kurstaki (Adang et al. (1985) Gene 36:289-300) and var. israelensis - (Waalwijk et al. (1985) Nucl. Acids Res. 13:8207-8217) strains was used as a guideline in generating a consensus codon bias for the synthesis of an oligonucleotide.

The 27-mer oligonucleotide, end-labelled with [$\gamma$-$^{32}$P] ATP was used to screen presumed recombinant clones by in situ colony hybridization for the presence of the δ-endotoxin gene according to the method of R.M. Torczynski et al. (1984) Proc. Natl. Acad. Sci. 81:6451-6455. Hybridization (in 90 mM Tris-HCl,pH 8.0, 5x Denhardt's solution, 900 mM NaCl, 6 mM $Na_2$EDTA, 0.1% SDS and 250 µg/ml heat-denatured tRNA) was carried out at room temperature for 18h using $10^6$ cpm/10ml/filter. Filters were repeatedly washed at room temperature with a solution containing 90 mM Tris-HCl, pH 8.0, 900 mM NaCl, 6 mM $Na_2$EDTA and 0.5% SDS prior to a final wash at 37° C for 5 min. When probed with the $^{32}$P-labelled oligonucleotide specific to the toxin gene of B. thuringiensis var. tenebrionis, 14 colonies were found to exhibit specific hybridization. Bioassays as described in example 3 were performed on extracts of these 14 strains and one, E. coli NSBP544, was toxic to Colorado Potato Beetle larvae.

Example 2: Preparation of toxic subclones of the B. thuringiensis var. tenebrionis gene

The relative locations of various restriction enzyme sites in the cloned DNA fragment containing the toxin gene of var. tenebrionis were determined by single and multiple digestions. The recombinant plasmid, pNSBP544, containing a 5.9 kb BamHI fragment inserted in the pUC12 vector, has multiple restriction endonuclease sites as schematically illustrated in Figure 1A.

To locate the crystal toxin gene within the cloned DNA segment (namely, pNSBP544), the 5.9 kb BamHI insert was transferred to the vector pIC20H (J. Lawrence-Marsh et al. (1984) Gene 32:481-485) using established methods (L. Marsh et al. (1984) Gene 32:481-485). Subcloning was carried out in E. coli strain HB101 using the plasmid vector pIC20H. Subclones were constructed by deletion of specific fragments. Subclones were used to partially define the coding region of the crystal protein. The E. coli 544 HindIII subclone, shown as a partial restriction map in Figure 1B, was constructed by cloning the 3.0 kb HindIII

fragment of pNSBP544 into pIC20H.

The subclone p544Pst-Met5 was constructed as a deletion derivative of the B. thuringiensis var. tenebrionis crystal protein gene. The 2.9 kb BamHI-PstI fragment of pNSBP544 was removed to delete the N-terminal 46 amino acid residues of the crystal protein. The p544PstSC was then digested with SphI and PctI and ligated with the linker segment described in Figure 1D to restore the Met codon in the B. thuringiensis var. tenebrionis sequence prior to the PstI site. In pIC20H reinitiation is required to use the Met of the modified B. thuringiensis var. tenebrionis sequence. Figure 2 describes the amino acid sequence of the crystal protein derived from the crystal protein gene of B. thuringiensis var. tenebrionis. When the sequence coding for the initial 46 amino acids was deleted from the N-terminus, the translation start ATG codon was also deleted. As shown in Figure 1C and 1D, a linker was built to restore a codon for a Met residue in the B. thuringiensis var. tenebrionis sequence prior to the Pst1 site, as well as to provide a bacterial ribosome binding site. Positive E. coli transformants were identified with end-labelled linker molecule.

Example 3: Expression of the var. tenebrionis δ-endotoxin gene in E. coli

E. coli cells that were transformed with the DNA fragments from B. thuringiensis var. tenebrionis and that tested positive in the in situ hybridization assay were grown individually in 100 ml of L-broth at 37°C for 18h. The proteins produced by these recombinant clones were screened for the presence of δ-endotoxin protein, the translation product of the δ-endotoxin gene. To assay for the presence of δ-endotoxin protein synthesized from the inserted B.thuringiensis var. tenebrionis gene, two criteria were established. The translation protein product must (1) show toxicity to insect larvae and (2) react with crystal toxin specific antibody.

The biological activity of the recombinant clones that showed specific hybridization to the oligomeric probe was tested by treating neonate larvae of Colorado potato beetle (Leptinotarsa decemlineata) with the total protein extracts obtained from these clones. About 5 mg of total protein from each clone was spread onto individual potato leaflets and fed to the larvae. Each clone was tested in duplicate including 5 larvae per sample. Leaves treated with buffer alone and leaves treated with 5 mg of total protein from an E. coli strain containing only pUC12 plasmid were included as controls. The amount of protein bound to the leaves was measured by rinsing the leaves with water and estimating the protein content of the rinse water. Insect mortality was recorded after two days.

In addition, the recombinant clones showing specific hybridization to the oligomeric probe were tested for activity against corn rootworms. Field corn was prepared by washing with bleach and soap. The kernels were allowed to germinate before being used for experimentation. Extracts containing B.thuringiensis var. tenebrionis protein, along with suitable controls, were incubated with germinating corn kernels. Into each 7 cm petri dish lined with moistened filter paper, 10 treated kernels and 10 neonate larvae were added.

The presence of endotoxin protein was also tested by immunoblot analysis. E. coli clones were grown overnight in 100 ml L-broth. Total protein extracts from the recombinant clones that tested positive in the in situ hybridization assays were prepared as described by Herrnstadt et al. (1986) Bio/Technology 4:305-308. About 100 µg of total protein from each sample was subjected to electrophoresis on a 10% (w/v) polyacrylamide gel in the presence of 0.1% (w/v) SDS. After electrophoresis, proteins were transferred to a nitrocellulose membrane filter and Western blot analysis of the membrane-bound proteins was performed by incubating the filter with a mouse antiserum specific to the 65 kDa crystal protein of B. thuringiensis var. tenebrionis. Membrane-bound proteins showing specific binding to the crystal protein antiserum were detected by treatment with a secondary, rabbit antimouse antiserum. The specific binding was detected by the utilization of $^{125}$I-Protein A.

Of the 14 recombinant clones found to exhibit specific hybridization when probed with the 27-mer oligonucleotide probe, only one recombinant clone, pNSBP544, was toxic to the larvae of Colorado potato beetle (illustrated in Table 2 below). The specific toxicity of the isolated crystal protein to induce 50% mortality was found to be approximately 0.18 µg/cm$^2$. Similar bioactivity values were obtained for the recombinant expression product using either the 5.9 kb BamHI fragment or the HindIII subclone.

TABLE 2

EFFECT OF INSECTICIDAL PROTEIN ON L. DECEMLINEATA LARVAE

| Treatment Activity | Dosage ($\mu g/cm^2$) | | |
|---|---|---|---|
| | total protein[a] | insecticidal protein[b] | percent mortality[c] |
| Buffer | | 0 | 5 |
| E. coli pIC20H | 36 | 0 | 0 |
| | 3.6 | 0 | 0 |
| E. coli pIC20H + IP[d] | 36 | 1800 | 80 |
| | 3.6 | 180 | 47 |
| | 0.36 | 18 | 23 |
| | 0.036 | 1.8 | 3 |
| E. coli pNSBP544 | 36 | 1800 | 78 |
| E. coli 544Bam(+)SC | 36 | 1800 | 76 |
| | 3.6 | 180 | 43 |
| | 0.36 | 18 | 13 |
| E. coli 544Bam(-)SC | 36 | 180 | 42 |
| | 3.6 | 18 | 3 |
| | 0.36 | 1.8 | 7 |
| E. coli 544HindSC | 36 | 1800 | 79 |

a Leaves treated with 3 mg protein/ml contained an estimated 36 $\mu g$ protein/$cm^2$.

b The amount of insecticidal protein (IP) was estimated by ELISA analysis.

c Mortality was scored two days after applying L. decemlineata larvae. These results are the average data of at least two trials with a minimum of 10 larvae/trial.

d E. coli pIC20H + IP was obtained by adding solubilized IP to crude E. coli extracts.

The cloned B. thuringiensis var. tenebrionis gene, as well as the crystal toxin extracted from B. thuringiensis var. tenebrionis spore/crystal mixtures, were also effective in causing mortality to western corn rootworm as is illustrated in Table 3 below.

TABLE 3

EFFECT OF INSECTICIDAL PROTEIN ON WESTERN CORN ROOTWORM (DIABROTICA VIRGIFERA VIRGIFERA)

| Treatment | No. Alive | No. Dead | Percent Recovery |
|---|---|---|---|
| Extract from Btt[a] crystals/spores[b] | 2 | 5 | 70 |
| " | 4 | 2 | 60 |
| " | 1 | 5 | 60 |
| Water control | 10 | 0 | 100 |
| " | 8 | 0 | 80 |
| " | 9 | 0 | 90 |
| Extract from recombinant Btt clones[c]: | | | |
| with BamHI fragment | 2.0 | 6.3 | 83.3 |
| with Hind111 fragment | 0.3 | 7.3 | 76.7 |
| Water control | 9.3 | 0.0 | 93.3 |
| Buffer control | 7.7 | 0.3 | 80.0 |
| Vector control | 7.0 | 0.7 | 76.7 |

[a] B. thuringiensis var. tenebrionis

[b] A crude extract from spores and crystals of B. thuringiensis var. tenebrionis was applied to germinating corn kernels. The protein level of the extract was not determined. After gentle shaking overnight, 10 neonate Western corn rootworm larvae were added to Petri dishes, each of which had 5 kernels. There were 3 replicates and a water control. After 6 days the larvae were recovered.

[c] Extracts from E. coli/Btt clones, E. coli/vector, buffer and water control were applied to germinating corn kernels and incubated for 30 min with gentle shaking. Into each 7 cm Petri dish lined with moistened filter paper, 10 neonate larvae were added. The results are averages for 3 replicates.

Table 3 indicates that the B. thuringiensis var. tenebrionis toxin, prepared by both extraction and recombinant procedures, caused mortality to Western corn rootworm. In similar bioassays, Southern corn rootworms were found to be less sensitive to the toxin expressed by the B. thuringiensis var. tenebrionis gene or to protein extract from B. thuringiensis var. tenebrionis spore/crystal mixtures.

Immunoblot analysis of the total soluble proteins from the 14 recombinant clones that hybridized with the 27-mer probe corroborated the bioassay results and also showed that only E. coli NSBP544 produced proteins that bound specifically to the 65 kDa crystal protein antiserum (illustrated in Figure 3A). In addition, immunospecific binding was also obtained with the E. coli 544HindIII subclone containing the 3.0 kb HindIII fragment harboring the δ-endotoxin gene from B. thuringiensis var. tenebrionis.

Immunoblot analysis of purified crystal inclusions of B. thuringiensis var. tenebrionis revealed the presence of a major crystal antigen of Mr 65 kDa (Figure 3, lane 2). When a higher concentration of the crystal protein sample was subjected to electrophoresis, the presence of additional minor peptide components of Mr 73 kDa and 56 kDa was revealed (Figure 3, lane 1). Sporulating cells of var. B. thuringiensis - (stage III-IV), on the other hand, were found to produce a major crystal antigen of Mr 73 kDa and a minor component of Mr 65 kDa (Figure 3, lane 3). Analysis of the total soluble protein of E. coli NSBP544 showed that this clone produced both 65 kDa and 73 kDa crystal antigens (Figure 3, lane 5). Although both peptides were present irrespective of the orientation of the 5.9 kb BamHI insert with respect to the lacZ gene of the vector plasmid, the concentration of both antigenic peptides was greatly reduced (approximately 10 times) in the E. coli 544Bam(-)SC extract (i.e., in which the orientation of the insert was opposite to the lacZ promoter of the vector; Figure 3, lane 6). The concentration of the crystal antigens in E. coli NSBP544 was estimated to be approximately 5% and in Bam(-) subclone to be approximately 0.5% of the total protein. In addition, the subclone E. coli 544HindSC, which contains the crystal protein gene flanked by 588 nucleotides at the 5′ end and 480 nucleotides at the 3′ end, expresses the 73 kDa and the 65 kDa peptides at a level equivalent to the parental E. coli pNSBP544 (Figure 3, lane 7). When 46 amino acids were deleted from the N-terminal end of the toxin gene, recombinant plasmids expressed predominantly the 65 kDa polypeptide which corresponds to the 597 amino acid polypeptide encoded within the open reading frame extending from nucleotide approximately 982 to 2775 (illustrated in Figure 2). This 65 kDa expression product is homogeneous as judged by HPLC and differs from the polypeptides comprising the heterologous (on HPLC) 65 kDa crystal isolated from B. thuringiensis var. tenebrionis. Any natural polypeptide isolated as the 65 kDa crystal protein most probably represents a proteolytically processed derivative of the larger 73 kDa crystal protein, whereas the recombinant polypeptide expression product is a uniform protein having an N-terminal methionine. In Figure 3B, lanes 2 and 3 compare the expression products when the coding region of the var. tenebrionis toxin gene is intact (lane 2) and when it is shortened by 46 nucleotides at the N-terminal end of the coding region (lane 3). Although modification/deletion at the N-terminal region of the B. thuringiensis var. tenebrionis crystal toxin gene did not compromise the expression of a crystal toxin polypeptide, even small (not greater than four or five amino acids) deletions at the C-terminus resulted in greater than ten fold loss of insecticidal activity.

Example 4: Nucleotide sequencing of the δ-endotoxin gene from B. thuringiensis var. tenebrionis

DNA sequencing was performed according to the chemical method of Maxam and Gilbert (1980) Meth. Enzymol. 65:499-559. The mapping of the 5′ end of the crystal toxin RNA transcripts was carried out using mung bean nuclease transcript mapping. A 737 bp PstI/HindIII fragment (end-labelled at the PstI terminus) was used as the probe.

Total RNA (30 µg each) isolated from logarithmic and stationary phase E. coli NSBP544 cells and from early stationary (2h after the onset of stationary phase, stage II of sporulation) and mid-stationary (71h into stationary phase, stage III-IV). B. thuringiensis var. tenebrionis cells were used in the mapping experiment.

Figure 2 shows the nucleotide sequence for positions 601 to 3000 of the 3.0 kb HindIII fragment of pNSBP544. An open reading frame extends from nucleotide 841 to 2775. This open reading frame encodes a peptide of 644 amino acids corresponding to a molecular weight of 73,119 Da. The methionine codon at position 841 is most likely the initiation methionine because of a potential ribosome binding site (with a calculated free energy of base pairing of -21.3 kcal to the 16S rRNA of B. subtilis) prior to the ATG at nucleotides 823-832. The deduced amino acid composition of the cloned crystal protein shows good agreement with the reported composition of var. tenebrionis (K. Bernhard (1986) FEMS Microbol. Lett. 33:261-265).

The 5′ end of the crystal protein RNA transcript in early and mid-sporulating stages of B. thuringiensis var. tenebrionis was mapped. A single start site located 130 bp upstream from the translational initiator ATG codon is used by var. tenebrionis during both early and mid-sporulation stages. A single transcriptional start site has also been reported for the 28 kDa gene of B. thuringiensis var. israelensis (Waaljik et al. (1985), supra). In contrast,the crystal protein gene of var. kurstaki is initiated at two different sites during early and mid-sporulation but at a single site in logarithmic and stationary E. coli cells (Wong et al. (1983) J. Biol. Chem. 258:1960-1967). The region proximal to the RNA start showed no significant homologies to B. subtilis consensus -10 and -35 promoter regions. Some homology, 5 base pairs out of 8 for the -35 region, GAATGATT, and 3 out of 8 for the -10 region, GTATAAAT, (underlined bases indicate identity), is present to the PB1 promoter of var. israelensis and the BT1 promoter of var. kurstaki. The var. tenebrionis gene has an A+T rich region (Figure 2) upstream from the site of RNA initiation. Similar A+T rich regions are located prior to the israelensis and kurstaki genes. Analysis of crystal protein gene transcripts in E. coli pNSBP544 using mung bean nuclease mapping indicates that transcription starts approximately 170 bp upstream from the initiation ATG. A consensus -10 E. coli promoter region (TATAAT) is located near this start.

## Example 5: Comparisons of crystal toxin gene and protein amino acid sequences from different subspecies of B. thuringiensis

Southern blot hybridization techniques were used to ascertain homology among different DNA samples. Chromosomal and plasmid DNA of various B. thuringiensis strains as well as E. coli recombinant strains were separated on a 0.6% agarose gel (Figure 4, panel A) by the modified slot-lysis method of Eckhardt (1978, Plasmid 1:584-588) and transferred to a nitrocellulose membrane filter. Using purified, nick-translated 0.7 kb internal EcoRI fragment of pNSBP544 as a probe, DNA-DNA hybridization experiments were carried out in 900 mM NaCl, 900 mM NaCitrate, 10 mM $Na_2EDTA$, 5X Denhardt's solution, 0.5% SDS and $10^6$ cpw/10ml/filter at 65° C for 18h. The filters were washed repeatedly at room temperature (in 450 mM NaCl, 450 mM NaCitrate and 0.5% SDS) prior to a final wash at 65° C for 90 min.

As shown in Figure 4, the internal 0.7 kb EcoRI fragment from the pNSBP544 gene hybridized only to var. tenebrionis DNA and not to B. thuringiensis var. kurstaki, var. thuringiensis or var. israelensis DNA. This indicates the coleopteran gene has little DNA homology with the lepidopteran active B. thuringiensis var. kurstaki HD73 or dipteran active B. thuringiensis var. israelensis genes.

The coleopteran gene has little DNA homology with the lepidopteran active B. thuringiensis var. kurstaki HD73 or dipteran active B. thuringiensis var. israelensis genes by the criteria of Southern hybridization. However, computer analysis indicates there is 50.2% amino acid homology between var. tenebrionis gene and the 5′ 1827 nucleotides of the 3537 nucleotides of var. kurstaki HD73 gene (Figure 5A). The hybridization of pNSBP544 probe (but not the internal 0.7 kb EcoRI fragment probe) to var. kurstaki strains may be due to this homology at the 5′ region. When the amino acid sequences of these two proteins are aligned, all matches occur between the complete var. tenebrionis protein and the N-terminal 609 amino acids of the HD73 protein. This is the region of the HD73 protein that contains the active toxin. Figure 5 shows the alignment of the var. tenebrionis gene product against the first 609 amino acids of the HD73 gene. Overall, there is 22.7% identity in this region; from amino acids 106-382 there is 33.0% homology and from amino acids 451-644 there is 22.4% homology. As seen in Figure 5, after a 33 amino acid gap is inserted at the start of the HD73 sequence, residues 67-94 of the var. tenebrionis protein align with residues 35-70 of the HD73 protein. These regions have a similar predicted hydropathy value (Figure 5B). Also, two shorter regions, amino acids 273-285 and 451-474, have very similar predicted hydropathy values. The most notable difference in toxin sequence between var. tenebrionis and kurstaki HD73 is the hydrophilic region from amino acid 423-445 of the var. tenebrionis protein which is absent in the HD73 protein. Further comparisons of these genes will lead to a strategy that can direct the modification of the activity of these insecticidal proteins. The proteins coded by these genes have similar insecticidal nature but different host range - properties that are encoded by specific DNA sequences.

## Example 6: Temporal regulation of expression of the crystal protein in B. thuringiensis var. tenebrionis

An overnight culture of B. thuringiensis var. tenebrionis that was grown at 30° C with shaking in supplemental spizizen's salts (SPY) medium was diluted 10-fold with fresh medium. This culture was grown at 37° C with shaking through two logarithmic growth transfers. A 100-fold dilution of this synchronized culture was made in 2 x 600 ml fresh medium. The growth rate of this culture was monitored by spectrometry at 600 nm. Two samples (30 ml each) of this culture were drawn at various growth stages. Protein extract was prepared from one sample and total RNA was extracted from another.

Purified RNA samples were dried under vacuum and resuspended in loading buffer (20mM) phosphate buffer, pH 6.8, 5mM $Na_2EDTA$, 25% formamide, 3% formaldehyde and 10% glycerol). The samples were denatured at 60° C for 10 min and electrophoresed on a 1% agarose gel at 1 V/cm for 16h. Electrophoresed RNA was transferred to Zetabind membrane filter (CUNO Filtration Products, Meriden, CT) and Northern blot was performed. A 0.7 kb internal EcoRI fragment ($^{32}P$-labeled by nick-translation) of B. thuringiensis var. tenebrionis crystal protein gene was used as a probe for the detection of crystal protein specific transcript. The protein extracts were subjected to SDS-polyacrylamide gel electrophoresis and antigenic peptides were detected by immunoblot analysis. The primary mouse antiserum was prepared against the 65 kDa crystal protein of B. thuringiensis var. tenebrionis. A goat-antimouse antibody conjugated to alkaline phosphatase was used as the secondary antiserum. Antigenic peptides were detected by a colorimetric assay specific for alkaline phosphatase.

The expression of the crystal protein in most B. thuringiensis strains begins at mid-sporulation stages and it is generally believed that the expression of all crystal protein genes is under stringent temporal control. In the case of B. thuringiensis var. tenebrionis; however, a 2.2 kb RNA transcript specific for the crystal protein gene was detected in logarithmically growing cells (Fig. 6, lane 1). The level of expression of this transcript increased dramatically (Fig. 6, lanes 2 and 3) in early stationary cells (i.e., 2h after the onset of stationary phase, $t_2$; this stage is generally referred to as sporulation stage 1) and was maintained at this level through mid-stationary phase ($t_7$, sporulation stage 111-IV). Correspondingly, detectable levels of the crystal antigen appeared in mid-log cells (Fig. 7B, lane 1) followed by its accumulation to maximal levels in early- and mid-stationary cells (Fig. 7B, lanes 2-4). This unique pattern of expression of the crystal protein gene of B. thuringiensis var. tenebrionis may be conferred by its promoter region. Table 4 below shows a comparison of -10 and -35 regions of the crystal protein genes of B. thuringiensis var. tenebrionis and other B. thuringiensis varieties. The B. thuringiensis varieties in which the expression of their crystal protein genes begins during mid-sporulation stages and reaches maximal levels at later stages were observed to contain highly conserved promoter regions. However, the promoter of the crystal protein gene of B. thuringiensis var. tenebrionis shares very little homology to that of other B. thuringiensis varieties. Shivakumar et al. (1986) (J. Bacteriol. 166:194-204) have observed vegetative expression of the crystal protein in a recombinant Bacillus subtilis strain containing the 6.6 kb crystal gene of B. thuringiensis var. kurstaki Hd1 dipel. However, the present invention documents the first example of vegetative cell expression of the crystal protein gene in any B. thuringiensis strain.

TABLE 4

COMPARISON OF THE PROMOTER SEQUENCES OF THE TOXIN GENES

| Toxin Gene | -35 | -10 |
|---|---|---|
| B. thuringiensis var. kurstaki (Br 1) | GCATTTTT | CATATGTT |
| B. thuringiensis var. israelensis (PB1) | GCATCTTT | CATAGAAT |
| B. thuringiensis var. tenebrionis | GAATGATT | GTATAAAT |

Bases underlined by solid lines indicate identity between all three toxin genes. Bases underlined by dotted lines indicate homology between PBI and var. tenebrionis promoters.

Results from immunoblot analysis indicated that in vegetative B. thuringiensis var. tenebrionis cells, the crystal protein was produced as a 73 kDa antigen (Fig. 7, lane 2). In early sporulation stage, an additional minor 65 kDa antigenic peptide appeared (Fig. 7, lane 3). The accumulation of this peptide increased through mid-sporulation stages. Proteases active at neutral pH have been reported to be associated with crystal inclusions of several B. thuringiensis strains. Nucleotide sequence analysis of the cloned crystal protein gene of B. thuringiensis var. tenebrionis revealed a 1932 bp open reading frame with a coding capacity of 73,119 daltons. Analysis of the deduced amino acid sequence revealed that a presumptive proteolytic cleavage site (arg-met) is located between residues 48 and 49. Proteolytic removal of this peptide from the N-terminal region of the 73 kDa crystal protein could result in the generation of a 65 kDa peptide which is the precise size of the additional crystal antigen that accumulates during later sporulation stages. Purified crystal protein preparations used as a control in this study were isolated at $t_{24}$ from a B. thuringiensis var. tenebrionis culture that was grown in SPY medium. This preparation contains trace amounts of 73 kDa peptide in addition to the primary 65 kDa crystal antigen (Fig. 7B, lane 5). However, crystal preparations isolated at $t_{24}$ from a B. thuringiensis var. tenebrionis culture that was grown in L-broth contained significant amounts (about 30%) of the 73 kDa peptide in addition to the 65 kDa crystal antigen. These results suggest that the actual ratio of the 65 and 73 kDa peptides present in purified crystal preparations is dependent upon the culture medium. In a culture medium that supports extensive sporulation (i.e., such as SPY medium), the amount of the 73 kda crystal protein is considerably reduced due to elevated proteolytic activities that accompany sporulation.

Example 7: Expression of a heterologous structural gene under the control of the B. thuringiensis var. tenebrionis crystal toxin gene promoter

This example describes the construction of a recombinant system for examining the expression of a heterologous structural gene under the control of the crystal toxin gene promoter from B. thuringiensis var. tenebrionis. Use of this system will enable one to obtain temporal regulation of expression of specific insecticidal antigens in spore forming bacilli.

The B. thuringiensis var. tenebrionis toxin gene promoter may be prepared from the 3.0 kb Hind111 fragment of pNSBP544 (example 2) by restriction endonuclease digestion (an example being digestion with XMnI and PstI as suggested by the restriction map presented in Figure 1B). The resultant piece of DNA comprising promoter region and approximately 138 bp 3′ of the ATG translation initiation codon may be shortened enzymatically (i.e., with exonuclease) at the 3′ end to give a fragment containing only the desired B. thuringiensis var. tenebrionis promoter. Alternatively, the B. thuringiensis var. tenebrionis toxin gene promoter may be chemically synthesized by utilizing the DNA nucleotide sequence disclosed in Figure 2 extending from nucleotides 600 to approximately 711.

Structural genes encoding insecticidal crystal proteins may be prepared from desired strains of B. thuringiensis according to published methods (see review by W. Reznikoff and L. Gold (1986). Procedures used in the construction of a recombinant B. thuringiensis var. tenebrionis crystal protein gene promoter - insecticidal protein structural gene fusion are standard and apparent to those of ordinary skill in the art. Components of the expression complex may be joined by any naturally occurring or constructed restriction sites convenient for in vitro manipulations. Incompatible ends of restriction fragments may be converted to blunt ends for ligation, or modified by the addition of linkers or adapters. The major consideration is that the sequences at the junctions maintain transcriptional and translational functionality.

B. thuringiensis var. tenebrionis crystal toxin gene promoter - heterologous structural gene construction may be introduced into conventionally-used strains of E. coli or other gram negative cells. Transformation of bacterial cells with exogenous DNA can be achieved in a number of ways known to those of ordinary skill experienced in the art. To examine the regulation pattern of expression controlled by the B. thuringiensis var. tenebrionis crystal protein gene promoter, expression of the recombinant gene construct may be monitored in spore-forming bacilli suitable for discerning temporal regulation in sporulating and vegetative cells. In addition to B. thuringiensis, a number of bacilli (including but not limited to B. popillae, B. larvae and B. sphaericus) produce parasporal inclusions. However, in most strains expression of the crystal toxin begins at mid-sporulation stages, whereas in B. thuringiensis var. tenebrionis expression of the crystal protein gene was detected in logarithmically growing cells.

## Claims

1. A cloned DNA sequence comprising a nucleotide sequence at least 75% homologous to a nucleotide sequence encoding amino acids 47 through 644 of the 73 kd toxin protein of Bacillus thuringiensis var. tenebrionis, said nucleotide sequence encoding an approximately 65 kd protein toxic to coleopteran insects.

2. The DNA segment of claim 1 wherein the nucleotide sequence encodes amino acids 47 through 644 of the 73 kd toxin protein of Bacillus thuringiensis var. tenebrionis.

3. A protein of approximately 65 kd having toxicity to coleopteran insects and having an N-terminal methionine.

4. The protein of claim 3 substantially free of contaminating proteins of Bacillus thuringiensis var. tenebrionis origin.

5. A cloned gene expressible in vegetative cells of Bacillus thuringiensis var. tenebrionis comprising a DNA segment having a nucleotide sequence at least 90% homologous to a promotor of a gene for a 73 kd protein of Bacillus thuringiensis var. tenebrionis toxic to coleopteran insects, and a coding region.

6. The gene of claim 5 wherein the coding region is DNA encoding an insect toxic protein.

7. The gene of claim 6 wherein the insect toxic protein is a protein of approximately 65 kd having toxicity to coleopteran insects and having an N-terminal methionine.

8. The gene of claim 6 wherein the insect toxic protein is a protein of Bacillus thuringiensis HD-1 toxic to lepidopteran insects.

9. The gene of claim 6 wherein the insect toxic protein is a protein of Bacillus thuringiensis var. kurstaki toxic to lepidopteran insects.

10. The gene of claim 6 wherein the insect toxic protein is a protein of Bacillus thuringiensis var. israeliensis toxic to dipteran insects.

11. A microorganism containing and expressing a gene comprising a cloned DNA sequence comprising a nucleotide sequence at least 75% homologous to a nucleotide sequence encoding amino acids 47-644 of the 73 kd toxin protein of Bacillus thuringiensis var. tenebrionis, said nucleotide sequence encoding an approximately 65 kd protein toxic to Coleopteran insects.

12. The microorganism of claim 11 wherein the microorganism is a gram-negative organism.

13. The microorganism of claim 11 wherein the microorganism is a gram-negative organism selected from the group Enterobacteriaceae,Pseudomonadaceae, Rhizobiaceae orBradyrhizobiaceae.

14. The microorganism of claim 11 wherein the microorganism is a variety of Bacillus thuringiensis.

15. A method of making a microorganism capable of expressing a protein toxic to Coleopteran insects comprising transforming a microorganism with a gene comprising a cloned DNA segment comprising a nucleotide sequence at least 75% homologous to a nucleotide sequence encoding amino acids 47-644 of the 73 kd toxin protein of Bacillus thuringiensis var. tenebrionis, said nucleotide sequence encoding an approximately 65 kd protein toxic to Coleopteran insects and a promoter capable of expressing said structural gene in the transformed microorganism.

16. The method of claim 15 wherein the promoter is a DNA segment at least 90% homologous to a promoter of a gene from the 73 kd protein of Bacillus thuringiensis var. tenebrionis toxic to Coleopteran insects.

17. The method of claim 15 wherein the microorganism is a gram-negative bacterium.

18. The method of claim 15 wherein the microorganism is a gram-negative organism selected from the group Enterobacteriaceae, Pseudomonadaceae, Rhizobiaceae, or Bradyrhizobiaceae.

19. The method of claim 15 wherein the microorganism is a variety of Bacillus thuringiensis.

Neu eingereicht / Newly filed
Nouvellement déposé

FIG. 1A.

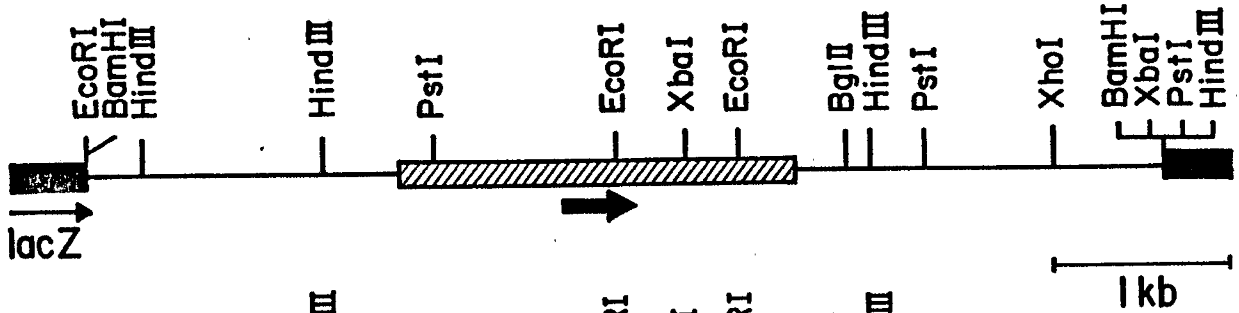


FIG. 1B.

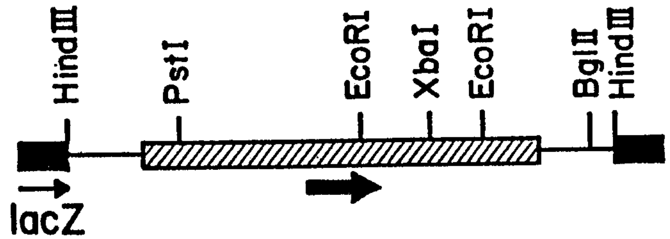


FIG. 1C.

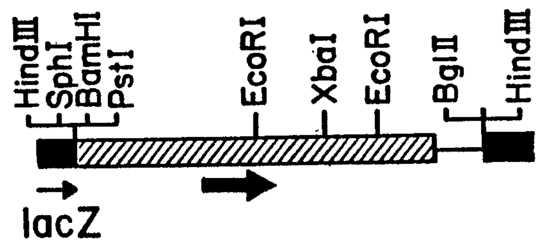


FIG. 1D

SD Met Thr Ala

CAGGATCCAACAATGACTGCA

GTACGTCCTAGGTTGTTACTG

SphI BAMHI PSTI

Neu eingereicht / Newly filed
Nouvellement déposé



# FIG. 2-I

Ec→ Bt→

```
601  TTAAATGAATATGTAAATATATTTATGATAAGAAGCGACTTATTTATAATCATTACATATTTTTCTATTGGAATGATTAAGATTCCAATAGAATAGTGTATAAATTATTTATCTTGAAAG

721  GAGGGATGCCTAAAAACGAAGAACATTAAAAACATATATTTGCACCGTCTAATGGATTTATGAAAAATCATTTTATCAGTTTGAAAATTATGTATTATGATAAGAAAGGGAGGAAGAAAA

841  ATGAATCCGAACAATCGAAGTGAACATGATACAATAAAAACTACTGAAAATAATGAGGTGCCAACTAACCATGTTCAATATCCTTTAGCGGAAACTCCAAATCCAACACTAGAAGATTTA
     MetAsnProAsnAsnArgSerGluHisAspThrIleLysThrThrGluAsnAsnGluValProThrAsnHisValGlnTyrProLeuAlaGluThrProAsnProThrLeuGluAspLeu

961  AATTATAAAGAGTTTTTAAGAATGACTGCAGATAATAATACGGAAGCACTAGATAGCTCTACAACAAAAGATGTCATTCAAAAAGGCATTTCCGTAGTAGGTGATCTCCTAGGCGTAGTA
     AsnTyrLysGluPheLeuArgMetThrAlaAspAsnAsnThrGluAlaLeuAspSerSerThrThrLysAspValIleGlnLysGlyIleSerValValGlyAspLeuLeuGlyValVal

1081 GGTTTCCCGTTTGGTGGAGCGCTTGTTTCGTTTTATACAAACTTTTTAAATACTATTTGGCCAAGTGAAGACCCGTGGAAGGCTTTTATGGAACAAGTAGAAGCATTGATGGATCAGAAA
     GlyPheProPheGlyGlyAlaLeuValSerPheTyrThrAsnPheLeuAsnThrIleTrpProSerGluAspProTrpLysAlaPheMetGluGlnValGluAlaLeuMetAspGlnLys

1201 ATAGCTGATTATGCAAAAAATAAAGCTCTTGCAGAGTTACAGGGCCTTCAAAATAATGTCGAAGATTATGTGAGTGCATTGAGTTCATGGCAAAAAAATCCTGTGAGTTCACGAAATCCA
     IleAlaAspTyrAlaLysAsnLysAlaLeuAlaGluLeuGlnGlyLeuGlnAsnAsnValGluAspTyrValSerAlaLeuSerSerTrpGlnLysAsnProValSerSerArgAsnPro

1321 CATAGCCAGGGGCGGATAAGAGAGCTGTTTTCTCAAGCAGAAAGTCATTTTCGTAATTCAATGCCTTCGTTTGCAATTTCTGGATACGAGGTTCTATTTCTAACAACATATGCACAAGCT
     HisSerGlnGlyArgIleArgGluLeuPheSerGlnAlaGluSerHisPheArgAsnSerMetProSerPheAlaIleSerGlyTyrGluValLeuPheLeuThrThrTyrAlaGlnAla

1441 GCCAACACACATTTATTTTTACTAAAAGACGCTCAAATTTATGGAGAAGAATGGGGATACGAAAAAGAAGATATTGCTGAATTTTATAAAAGACAACTAAAACTTACGCAAGAATATACT
     AlaAsnThrHisLeuPheLeuLeuLysAspAlaGlnIleTyrGlyGluGluTrpGlyTyrGluLysGluAspIleAlaGluPheTyrLysArgGlnLeuLysLeuThrGlnGluTyrThr

1561 GACCATTGTGTCAAATGGTATAATGTTGGATTAGATAAATTAAGAGGTTCATCTTATGAATCTTGGGTAAACTTTAACCGTTATCGCAGAGAGATGACATTAACAGTATTAGATTTAATT
     AspHisCysValLysTrpTyrAsnValGlyLeuAspLysLeuArgGlySerSerTyrGluSerTrpValAsnPheAsnArgTyrArgArgGluMetThrLeuThrValLeuAspLeuIle

1681 GCACTATTTCCATTGTATGATGTTCGGCTATACCCAAAAGAAGTTAAAACCGAATTAACAAGAGACGTTTTAACAGATCCAATTGTCGGAGTCAACAACCTTAGGGGCTATGGAACAACC
     AlaLeuPheProLeuTyrAspValArgLeuTyrProLysGluValLysThrGluLeuThrArgAspValLeuThrAspProIleValGlyValAsnAsnLeuArgGlyTyrGlyThrThr
```

Nouvellement déposé



1801 TTCTCTAATATAGAAAATTATATTCGAAAACCACATCTATTTGACTATCTGCATAGAATTCAATTTCACACGCGGTTCCAACCAGGATATTATGGAAATGACTCTTTCAATTATTGGTCC
PheSerAsnIleGluAsnTyrIleArgLysProHisLeuPheAspTyrLeuHisArgIleGlnPheHisThrArgPheGlnProGlyTyrTyrGlyAsnAspSerPheAsnTyrTrpSer

1921 GGTAATTATGTTTCAACTAGACCAAGCATAGGATCAAATGATATAATCACATCTCCATTCTATGGAAATAAATCCAGTGAACCTGTACAAAATTTAGAATTTAATGGAGAAAAAGTCTAT
GlyAsnTyrValSerThrArgProSerIleGlySerAsnAspIleIleThrSerProPheTyrGlyAsnLysSerSerGluProValGlnAsnLeuGluPheAsnGlyGluLysValTyr

2041 AGAGCCGTAGCAAATACAAATCTTGCGGTCTGGCCGTCCGCTGTATATTCAGGTGTTACAAAAGTGGAATTTAGCCAATATAATGATCAAACAGATGAAGCAAGTACACAAACGTACGAC
ArgAlaValAlaAsnThrAsnLeuAlaValTrpProSerAlaValTyrSerGlyValThrLysValGluPheSerGlnTyrAsnAspGlnThrAspGluAlaSerThrGlnThrTyrAsp

2161 TCAAAAAGAAATGTTGGCGCGGTCAGCTGGGATTCTATCGATCAATTGCCTCCAGAAACAACAGATGAACCTCTAGAAAAGGGATATAGCCATCAACTCAATTATGTAATGTGCTTTTTA
SerLysArgAsnValGlyAlaValSerTrpAspSerIleAspGlnLeuProProGluThrThrAspGluProLeuGluLysGlyTyrSerHisGlnLeuAsnTyrValMetCysPheLeu

2281 ATGCAGGGTAGTAGAGGAACAATCCCAGTGTTAACTTGGACACATAAAAGTGTAGACTTTTTTAACATGATTGATTCGAAAAAAATTACACAACTTCCGTTAGTAAAGGCATATAAGTTA
MetGlnGlySerArgGlyThrIleProValLeuThrTrpThrHisLysSerValAspPhePheAsnMetIleAspSerLysLysIleThrGlnLeuProLeuValLysAlaTyrLysLeu

2401 CAATCTGGTGCTTCCGTTGTCGCAGGTCCTAGGTTTACAGGAGGAGATATCATTCAATGCACAGAAAATGGAAGTGCGGCAACTATTTACGTTACACCGGATGTGTCGTACTCTCAAAAA
GlnSerGlyAlaSerValValAlaGlyProArgPheThrGlyGlyAspIleIleGlnCysThrGluAsnGlySerAlaAlaThrIleTyrValThrProAspValSerTyrSerGlnLys

2521 TATCGAGCTAGAATTCATTATGCTTCTACATCTCAGATAACATTTACACTCAGTTTAGACGGGGCACCATTTAATCAATACTATTTCGATAAAACGATAAATAAAGGAGACACATTAACG
TyrArgAlaArgIleHisTyrAlaSerThrSerGlnIleThrPheThrLeuSerLeuAspGlyAlaProPheAsnGlnTyrTyrPheAspLysThrIleAsnLysGlyAspThrLeuThr

2641 TATAATTCATTTAATTTAGCAAGTTTCAGCACACCATTCGAATTATCAGGGAATAACTTACAAATAGGCGTCACAGGATTAAGTGCTGGAGATAAAGTTTATATAGACAAAATTGAATTT
TyrAsnSerPheAsnLeuAlaSerPheSerThrProPheGluLeuSerGlyAsnAsnLeuGlnIleGlyValThrGlyLeuSerAlaGlyAspLysValTyrIleAspLysIleGluPhe

IR
2761 ATTCCAGTGAATTAAATTAACTAGAAAGTAAAGAAGTAGTGACCATCTATGATAGTAAGCAAAGGATAAAAAAATGAGTTCATAAAATGAATAACATAGTGTTCTTCAACTTTCGCTTTT
IleProValAsnEnd

IR
2881 TGAAGGTAGATGAAGAACACTATTTTTATTTTCAAAATGAAGGAAGTTTTAAATATGTAATCATTTAAAGGGAACAATGAAAGTAGGAAATAAGTCATTATCTATAACAAAATAACATTT

FIG. 2-2

Neu eingereicht / Newly filed
Nouvellement déposé

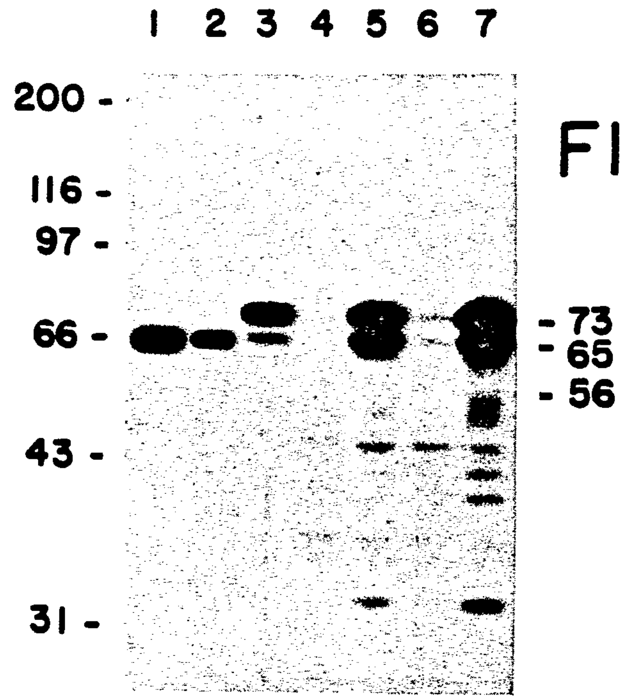


FIG. 3A

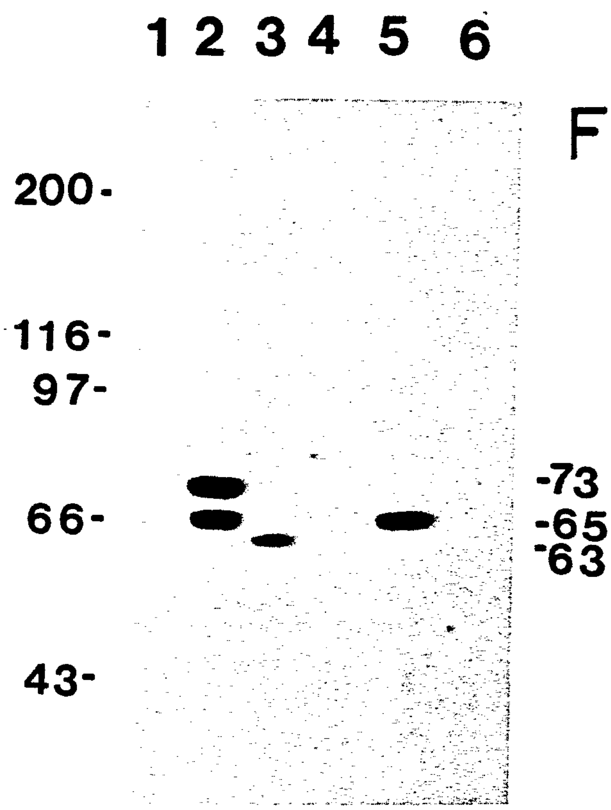


FIG. 3B

Neu eingereicht / Newly filed
Nouvellement déposé



FIG. 4A

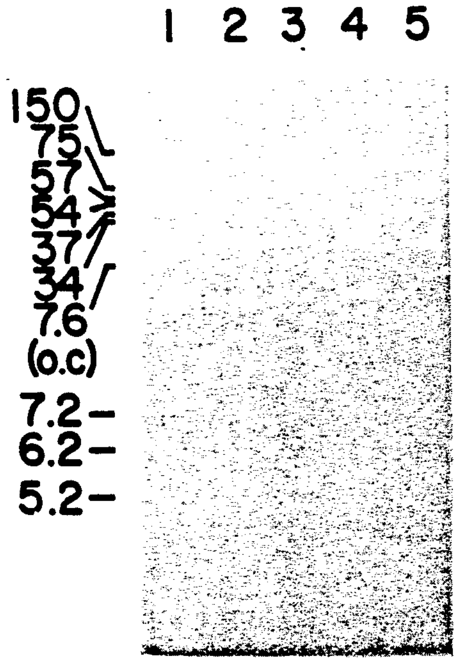


FIG. 4B

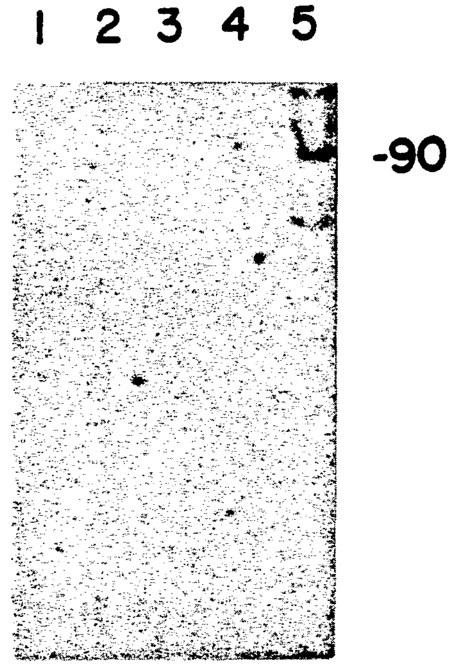


FIG. 4C

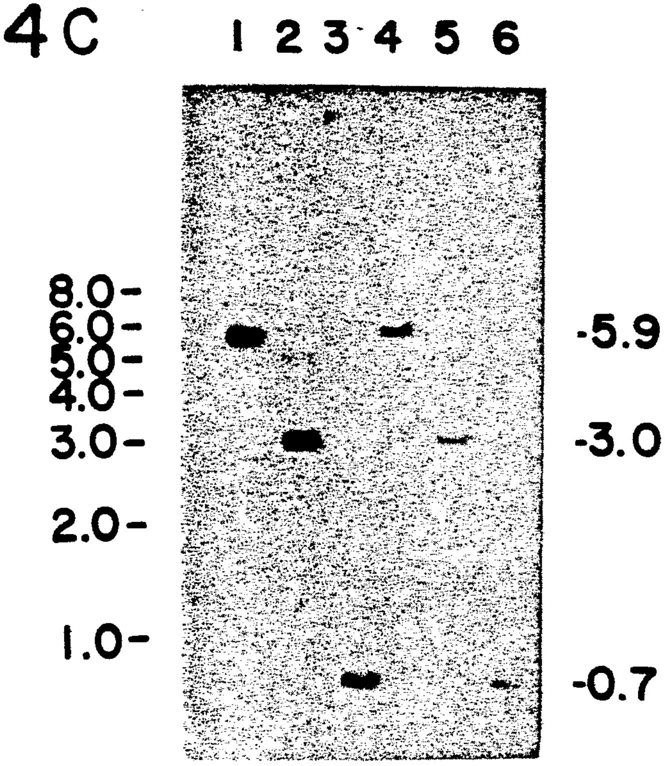

Neu eingereicht / Newly filed
Nouvellement déposé



FIG. 5 A.

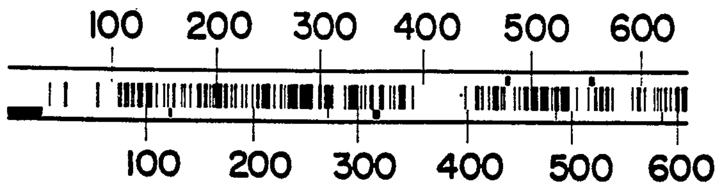


FIG. 5 B.

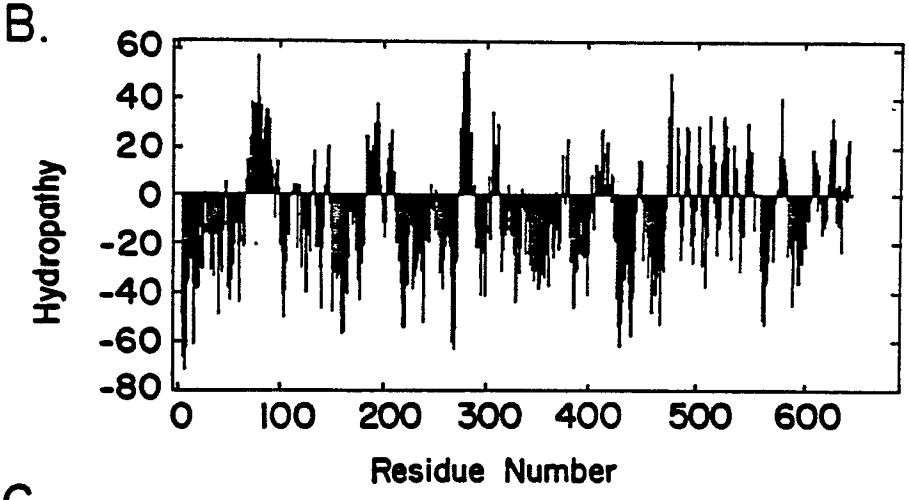


FIG. 5 C.

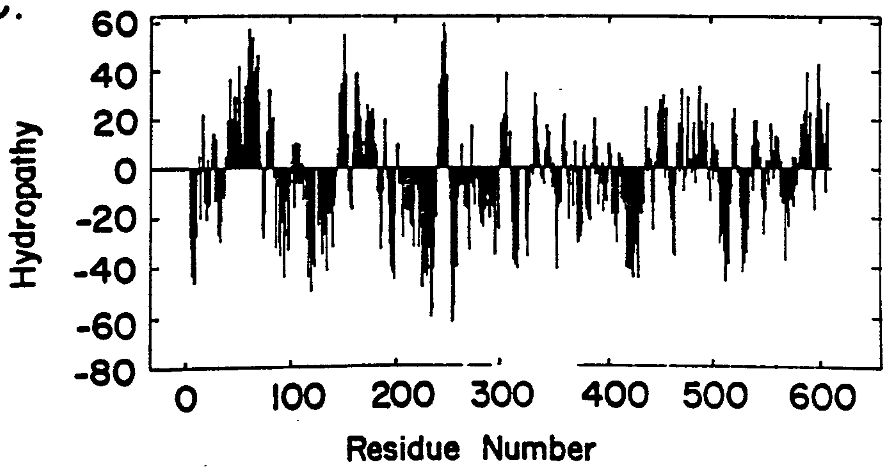

Neu eingereicht / Newly filed
Nouvellement déposé



FIG. 6

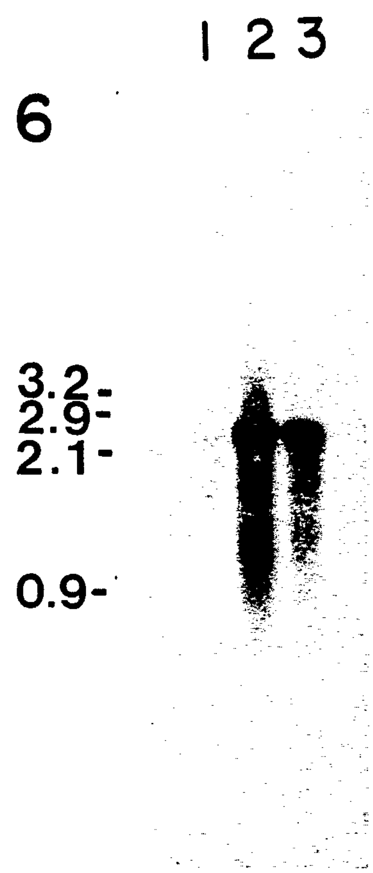


FIG. 7